# EUROPÄISCHE PATENTSCHRIFT

(11) **EP 1 632 262 B1**
(45) Veröffentlichungstag und Bekanntmachung des Hinweises auf die Patenterteilung: **22.06.2016**
(21) Anmeldenummer: 05019159.2
(22) Anmeldetag: 02.09.2005
(51) Int. Cl.: A61M 16/06

(54) **Vorrichtung zur Beatmung**
Respiratory device
Dispositif respiratoire

(30) Priorität: 03.09.2004 DE 102004043208
(43) Veröffentlichungstag der Anmeldung: 08.03.2006
(73) Patentinhaber: Weinmann Geräte für Medizin GmbH + Co. KG, 22525 Hamburg (DE)
(72) Erfinder: Frerichs, Arnold, 21614 Buxtehude (DE); Eifler, Martin, 25348 Glückstadt (DE); Feldhahn, Karl Andreas, 22761 Hamburg (DE)
(74) Vertreter: Patentanwälte Klickow & Partner Partnerschaftsgesellschaft mbB

(56) Entgegenhaltungen:
- EP-A- 1 205 205
- EP-A- 1 334 742
- WO-A-03/035156
- WO-A2-2004/021960
- US-A- 2 245 658

## Beschreibung

Die Erfindung betrifft eine Vorrichtung zur Beatmung, die als mindestens ein Teil eines Benutzerinterfaces ausgebildet ist, die eine Stirnstütze aufweist, die mit einer Verstellung zur Veränderung der Stirnstütze relativ zu einem Maskengrundkörper versehen ist und bei der die lösbare Arretierung eine lineare gerastete Feinverstellung mit einem Tragsteg umfaßt, der eine Sockelplatte der Stirnseite haltert, wobei an der Sockelplatte ein Stirnpolster befestigt ist, wobei sich der Tragsteg ausgehend von der Sockelplatte in eine dem Stirnpolster abgewandte Richtung erstreckt, wobei zur Führung des Tragsteges ein Schaft mit einem Tragprofil versehen ist, wobei die Arretierung mindestens eine Rastung umfasst und wobei die Rastung durch einen manuellen Druck von einem Gegenprofil trennbar angeordnet ist.

Derartige Benutzerinterfaces können in unterschiedlichen Ausführungsformen realisiert sein und dienen typischerweise dazu, von einem Beatmungsgerät oder einem Atemgas- oder Sauerstoffvorrat bereitgestelltes Atemgas an einen Patienten abzugeben. Derartige Benutzerinterfaces können beispielsweise als Sauerstoffbrillen oder als Atemmasken realisiert sein.

Atemmasken werden beispielsweise im Zusammenhang mit Beatmungsgeräten verwendet, um Atemgas zum Patienten zu leiten und eine Ableitung des ausgeatmeten Atemgases zu unterstützen. Die Atemmaske ist typischerweise über den Atemschlauch mit dem Beatmungsgerät verbunden.

Die Atemmasken werden häufig als ein modulares System ausgebildet, bei dem eine Vielzahl von einzelnen Systemkomponenten mechanisch zusammengefügt werden. Durch die einzelnen Funktionsmodule werden zum einen unterschiedliche Größen der Atemmasken und unterschiedliche Körpergestaltungen der jeweiligen Patienten abgedeckt, darüber hinaus können durch das Modulsystem auch unterschiedliche Funktionalitäten bereitgestellt werden.

Zur Gewährleistung einer sicheren Positionierung der Atemmaske im Bereich des Gesichtes eines Patienten sowie zur Abstützung und Übertragung von Kräften, die über einen Atemschlauch bzw. ein Schlauchanschlußstück in die Atemmaske eingeleitet werden, werden häufig Stirnstützen mit der Atemmaske verbunden. Typischerweise besitzen derartige Stirnstützen Höhen- und Abstandsjustierungen, um eine Anpassung an die vorliegende Gesichtsgeometrie eines Patienten vorzunehmen. Bekannt sind sowohl stufenlose Verstellmöglichkeiten als auch gerasterte Verstellmöglichkeiten, die eine Positionsveränderung in vorgegebenen Schritten ermöglichen.

Ein Nachteil sowohl der stufenlosen als auch der gerasterten Verstelleinrichtungen für die Stirnstützen besteht darin, dass die Vielzahl an möglichen Einstellvarianten einen Benutzer häufig überfordert und nach einer Demontage und Reinigung der Stirnstütze die ursprüngliche Abstandseinstellung nur schwer wieder reproduzierbar ist. Darüber hinaus weisen die bekannten Verstellmöglichkeiten den Nachteil auf, dass bei der unbeabsichtigten Einbringung von zu großen Kräften in die Stelleinrichtung die häufig aus Kunststoff bestehenden Bauteile zerbrechen und eine weitere Benutzung nicht mehr möglich ist, so dass eine kostenintensive Neubeschaffung erforderlich wird.

Die EP 1 334 742 A offenbart eine Vorrichtung zur Beatmung, die aus mehreren Teilen eines Benutzerinterfaces ausgebildet ist, wobei eine Stirnstütze vorgesehen ist, die mit mittels einer lösbaren Arretierung eine gerastete Feinverstellung zur Veränderung der Stirnstütze relativ zu dem Maskengrundkörper ermöglicht.

Aus der EP 1 205 205 A ist eine Vorrichtung zur Beatmung bekannt, die als Teil eines Benutzer-Interfaces ausgebildet ist. Zwei Komponenten des BenutzerInterfaces sind durch eine manuell lösbare Arretierung miteinander verbunden. Das Benutzer-Interface ist als eine Atemmaske ausgebildet, die eine Stimstütze aufweist.

In der WO 2004/021960 A2 wird ebenfalls eine Vorrichtung zur Beatmung beschrieben, die als Teil eines Benutzer-Interfaces ausgebildet ist. Auch hier sind mindestens zwei Komponenten des Benutzer-Interfaces durch eine manuell lösbare Arretierung miteinander verbunden. Ebenfalls ist das BenutzerInterface als Atemmaske ausgebildet, das eine Stirnstütze aufweist.

Aufgabe der vorliegenden Erfindung ist es daher, eine Vorrichtung zur Beatmung der einleitend genannten Art derart zu konstruieren, dass eine funktionelle, einfach zu bedienende und zugleich robuste Verstelleinrichtung bereitgestellt wird.

Diese Aufgabe wird erfindungsgemäß dadurch gelöst, dass ein Anschlag im Schaft der Begrenzung des Verstellweges des Tragsteges innerhalb der Feinverstellung dient und dass das Tragprofil in eine Führungsausnehmung des Tragsteges eingreift und dass die Rastung mit einer Einführanschrägung versehen ist und die Rastung mindestens eine Rastnase aufweist.

Die manuell lösbare Arretierung ermöglicht es, die zwei Komponenten in einer von einem Benutzer ausgewählten Positionierung zusammenzustecken und erforderlichenfalls zur Durchführung von Reinigungsvorgängen oder zu einer Positionsanpassung wieder voneinander zu trennen.

Eine Ausführungsform zur Anpassung einer Atemmaske an eine Gesichtskontur eines Patienten wird dadurch bereitgestellt, dass das Benutzerinterface als Atemmaske ausgebildet ist, die eine Stirnstütze aufweist, die mit einer Verstellung zur Veränderung der Stirnstütze relativ zu einem Maskengrundkörper versehen ist und bei der die lösbare Arretierung eine lineare gerastete Feinverstellung umfaßt.

Eine Anpassung an unterschiedliche Stirnkonturen eines Patienten kann dadurch erfolgen, dass sowohl eine Feinverstellung mit einer feinen Rastung als auch eine Grobverstellung in Form einer Umsteckbarkeit der Stirnstütze im Maskenkörper realisiert ist.

Eine einfach zu realisierende Grobverstellung wird dadurch bereitgestellt, daß die Stirnstütze wahlweise in jeweils mindestens einer von mehreren Steckpositionen im Maskenkörper als Grobverstellung positionierbar ist.

Zur Bereitstellung eines modularen Systems mit wahlweiser Verwendung einer Stirnstütze oder eines Abschlußelementes wird vorgeschlagen, daß der Maskengrundkörper einen Adapter zur Verbindung mit der Stirnstütze aufweist und daß der Adapter zur Aufnahme mindestens eines unterschiedlich zur Stirnstütze gestalteten Bauteiles ausgebildet ist.

Eine hohe Funktionalität wird dadurch bereitgestellt, daß eine Sockelplatte der Stirnstütze eine zentrierung zur Ausrichtung eines Stirnpolsters aufweist.

Eine einfache Adaptierbarkeit wird insbesondere dadurch unterstützt, daß mindestens die Grobverstellung oder die Feinverstellung durch einen manuellen Druck entriegelbar ausgebildet sind.

Ein einfaches Einführen in eine vorgewählte Positionierung wird dadurch unterstützt, daß die Arretierung mindestens eine Rastung mit einer Einführanschrägung umfaßt.

Eine Positionssicherheit wird auch bei einwirkenden mechanischen Belastungen kann dadurch unterstützt werden, daß die Rastung mindestens eine Rastnase aufweist.

Für die Durchführung von Positionsveränderungen erweist es sich als besonders vorteilhaft, daß die Rastung durch einen manuellen Druck von einem Gegenprofil trennbar angeordnet ist.

Eine einfache Bedienbarkeit wird dadurch unterstützt, daß zur Aufbringung von Bedienkräften gewölbt konturierte Bedienflächen verwendet sind.

Eine spritzgußtechnische Herstellung wird dadurch erleichtert, daß mindestens eines der Bauteile aus einem mit Versteifungsrippen versehenen dünnen Material ausgebildet ist.

Besonders geringe erforderliche manuelle Verstellkräfte werden dadurch erreicht, daß die gewölbten Bedienflächen Griffmulden mit einer Leitfunktion bereitstellen.

Zu einer hohen mechanischen Stabilität bei gleichzeitig einfach und mit hoher Qualität herstellbaren spritzgußtechnischen Bauteilen wird vorgeschlagen, daß mindestens eine Halterungsstruktur durch einen dünnwandigen dreidimensionalen Materialverlauf ausgebildet ist.

Bei einer Realisierung des Benutzerinterfaces als Atemmaske erweist es sich als vorteilhaft, daß die Grobverstellung im Bereich eines Überganges eines Schaftes der Stirnstütze zum Maskengrundkörper angeordnet ist.

Ebenfalls trägt es zu einem hohen Benutzungskomfort bei, daß die Feinverstellung im Bereich eines Überganges des Schaftes zur Sockelplatte angeordnet ist.

Eine weitere bevorzugte Ausführungsform ist darin zu sehen, daß die Grobverstellung einen im Bereich von Halterungsausnehmungen des Maskenkörpers umsteckbaren Schaft der Stirnstütze sowie eine durch federnde Befestigungslaschen bereitgestellte Arretierung umfaßt.

Eine weitere vorteilhafte Ausführungsvariante besteht darin, daß die Feinverstellung einen aus einer Führungsausnehmung des Schaftes herausziehbaren Tragsteg der Sockelplatte sowie eine durch Bedienlaschen bereitgestellte Arretierung umfaßt.

Eine harmonische Oberflächenkontur mit ineinander übergeleiteten Einzelflächen wird dadurch bereitgestellt, daß die Befestigungslaschen und die Bedienlaschen in einer Längsrichtung des Schaftes hintereinander angeordnet sind.

Die Erfindung wird im folgenden beispielhaft anhand der Figuren erläutert. Es zeigen:
- Fig. 1: ein als Nasalmaske ausgebildetes Patienteninterface in perspektivischer Ansicht,
- Fig. 2: eine Ansicht auf das Innere der Maske aus Fig. 1 von hinten,
- Fig. 3: eine Ansicht auf das Äußere der Maske aus Fig. 1 von vorne,
- Fig. 4: den Grundkörper der Maske aus Fig. 1 in perspektivischer Ansicht,
- Fig. 5: eine Seitenansicht eines mechanisch kodierten verbindungselementes,
- Fig. 6: eine perspektivische Darstellung des Maskengrundkörpers mit Blick auf ein Verbindungselement zur Aufnahme einer Stirnstütze,
- Fig. 7: eine Atemmaske mit einer in unterschiedlichen Positionen einsteckbaren Stirnstütze,
- Fig. 8: eine Seitenansicht der Anordnung gemäß Fig. 7,
- Fig. 9: die Anordnung gemäß Fig. 7 mit einer anderen Steckpositionierung der Stirnstütze,
- Fig. 10: eine Seitenansicht der Vorrichtung gemäß Fig. 9,
- Fig. 11: eine Atemmaske mit einer nochmals veränderten Positionierung der Stirnstütze,
- Fig. 12: eine Seitenansicht der Vorrichtung gemäß Fig. 11,
- Fig. 13: eine abgewandelte Ausführungsform zur veranschaulichung der Verwendung einer Grobverstellung sowie einer zusätzlichen Feinverstellung zur Verbindung von zwei Bauteilen des Patienteninterfaces,
- Fig. 14: die Anordnung gemäß Fig. 8 nach einer Verstellung der Position des Stirnpolsters,
- Fig. 15: die Anordnung gemäß Fig. 10 nach einer Verdrehung des Stirnpolsters,
- Fig. 16: eine perspektivische Darstellung der Anordnung gemäß Fig. 14,
- Fig. 17: eine Darstellung der Atemmaske zur Veranschaulichung einer Arretierung der Stirnstütze am Maskenkörper,
- Fig. 18: eine teilweise geschnittene Darstellung der Anordnung gemäß Fig. 17 bei einer Blickrichtung von hinten,
- Fig. 19: eine perspektivische Darstellung einer Stirnstütze,
- Fig. 20: eine Draufsicht auf die Stirnstütze gemäß Fig. 19,
- Fig. 21: eine Seitenansicht der Stirnstütze gemäß Fig. 19,
- Fig. 22: eine perspektivische Darstellung einer Stirnstütze zur Veranschaulichung der Realisierung einer Feinverstellung,
- Fig. 23: eine Draufsicht auf die Stirnstütze gemäß Fig. 22,
- Fig. 24: eine Seitenansicht der Stirnstütze gemäß Fig. 22,
- Fig. 25: eine Darstellung entsprechend Fig. 22 bei einer anderen Positionierung der Feinverstellung,
- Fig. 26: eine Draufsicht auf die Stirnstütze gemäß Fig. 25,
- Fig. 27: eine Seitenansicht der Stirnstütze gemäß Fig. 25,
- Fig. 28: eine vergrößerte Darstellung zur Veranschaulichung der Verstell- und Arretierungseinrichtung der Stirnstütze,
- Fig. 29: eine Darstellung der Verbindungseinrichtung gemäß Fig. 28 bei einer Blickrichtung von hinten,
- Fig. 30: eine Seitenansicht der Verbindungseinrichtung gemäß Fig. 29,
- Fig. 31: eine perspektivische Darstellung der Einrichtung gemäß Fig. 28,
- Fig. 32: eine Darstellung einer Halteeinrichtung für das Stirnpolster,
- Fig. 33: eine perspektivische Darstellung des Stirnpolsters,
- Fig. 34: eine perspektivische Darstellung des Stirnpolsters gemäß Fig. 33 bei einer Blickrichtung von hinten,
- Fig. 35: eine Darstellung des Stirnpolsters gemäß Fig. 33 in einem auseinandergenommenen Zustand,
- Fig. 36: eine perspektivische Darstellung der Halteeinrichtung gemäß Fig. 32 bei einer Blickrichtung von hinten,
- Fig. 37: eine Draufsicht auf die Halteeinrichtung gemäß Fig. 36,
- Fig. 38: eine perspektivische Darstellung der Einrichtung gemäß Fig. 32,
- Fig. 39: eine Draufsicht auf die Halteeinrichtung gemäß Fig. 38 bei einer Blickrichtung von oben,
- Fig. 40: eine Atemmaske mit einer positionierbaren Stirnstütze, bei der mindestens eine Positionsveränderung von einer Schwenkbewegung abgeleitet wird,
- Fig. 41: eine gegenüber der Darstellung in Fig. 40 abgewandelte Ausführungsform,
- Fig. 42: eine Atemmaske mit einer Stirnstütze, bei der eine Positionsveränderung der Stirnstütze durch eine Gleitbewegung entlang einer gekrümmten Führungsfläche abgeleitet wird,
- Fig. 43: eine weitere Ausführungsform mit einer verschwenkbar angeordneten Stirnstütze und
- Fig. 44: eine Atemmaske mit einer Stirnstütze, die entlang einer gekrümmten Führungsbahn verschieblich angeordnet ist.

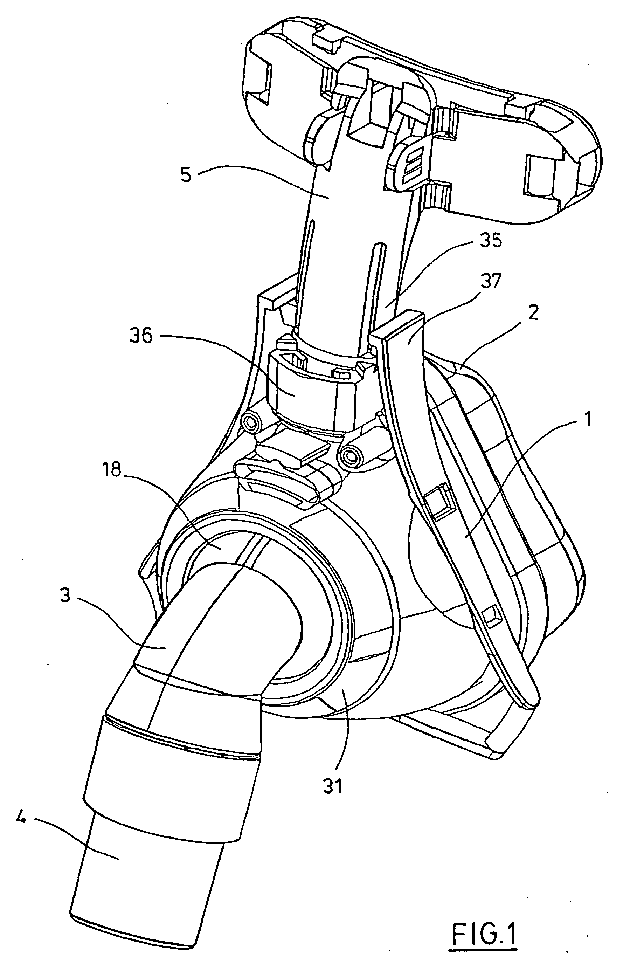

Fig. 1 zeigt ein als Nasalmaske ausgebildetes Patienteninterface, deren Maskenkörper (1) aus einem relativ festen Material gefertigt ist und die einen Maskenwulst (2) aufweist. Der Maskenwulst (2) dient zur Anlage am Gesicht eines nicht abgebildeten Patienten und gewährleistet die erforderliche Abdichtung. Über ein winkelförmig ausgebildetes Anschlußstück (3) ist der Maskenkörper (1) mit einer drehbeweglich gelagerten Hülse (4) verbunden, die zum Anschluß an einen nicht abgebildeten Atemgasschlauch dient. Zur Gewährleistung einer sicheren Positionierung der Atemmaske im Kopfbereich eines Patienten wird eine Stirnstütze (5) verwendet, die mit einem Schaft (35) in eine Halterung (36) des Maskenkörpers (1) eingeführt ist. Das Anschlußstück (3) und der Maskenkörper (1) sind über ein Kugelgelenk (18) miteinander verbunden. Das Kugelgelenk (18) ist von einem Sicherungsring (31) gelagert.

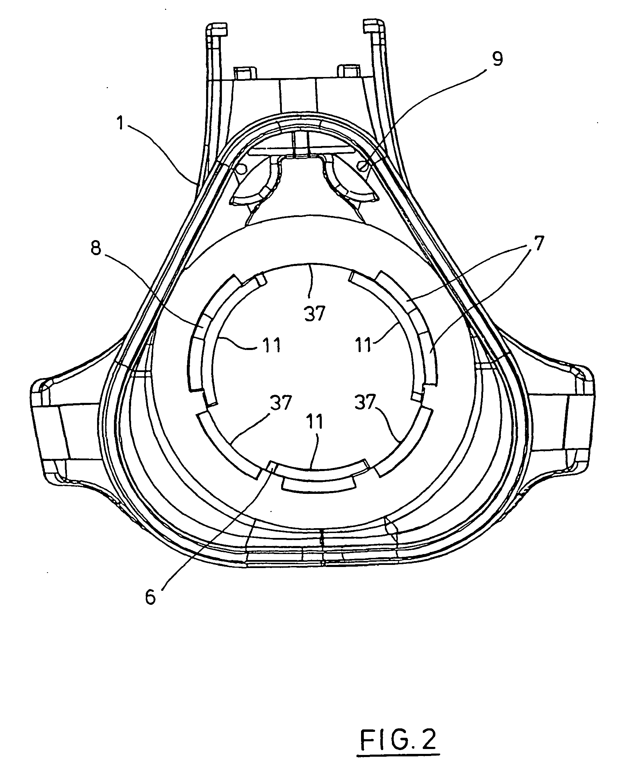

In Fig. 2 ist das Innere der Nasalmaske aus Fig. 1 bei einer Blickrichtung von innen in Richtung auf eine Aufnahme für das hier nicht abgebildete Kugelgelenk (18) dargestellt. Im oberen Bereich sind zwei Druckmeßstutzen (9) angeordnet. Strömungsöffnungen (7) führen zu nicht abgebildeten Ausatemspalten (14). Einführschrägen (6) sind für das erleichterte Einführen von Bajonettzähnen (26) eines nicht dargestellten Kugelkäfigs (24) vorgesehen. Die Bajonettzähne (26) bilden gemeinsam mit Rippen (11) eine mechanische Kodierung aus. Die Rippen (11) sind über Stege (8) mit dem Maskenkörper (1) verbunden.

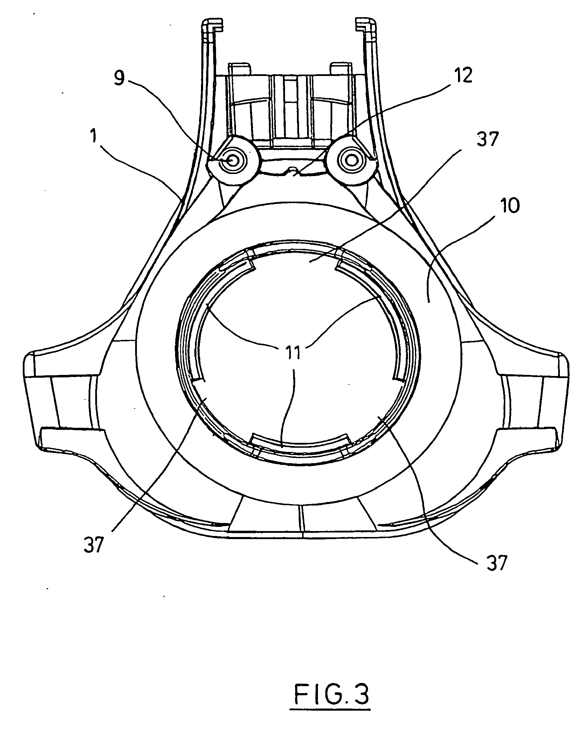

Fig. 3 gibt den Blick auf das Äußere der Maske aus Fig. 1 bei einer Blickrichtung von vorne frei. Eine Ausströmfläche (10) ist ringförmig ausgebildet. Mit Hilfe von Rippen (11) und einer verrastung (12) kann ein hier nicht abgebildeter Sicherungsring (31) angebracht und fixiert werden.

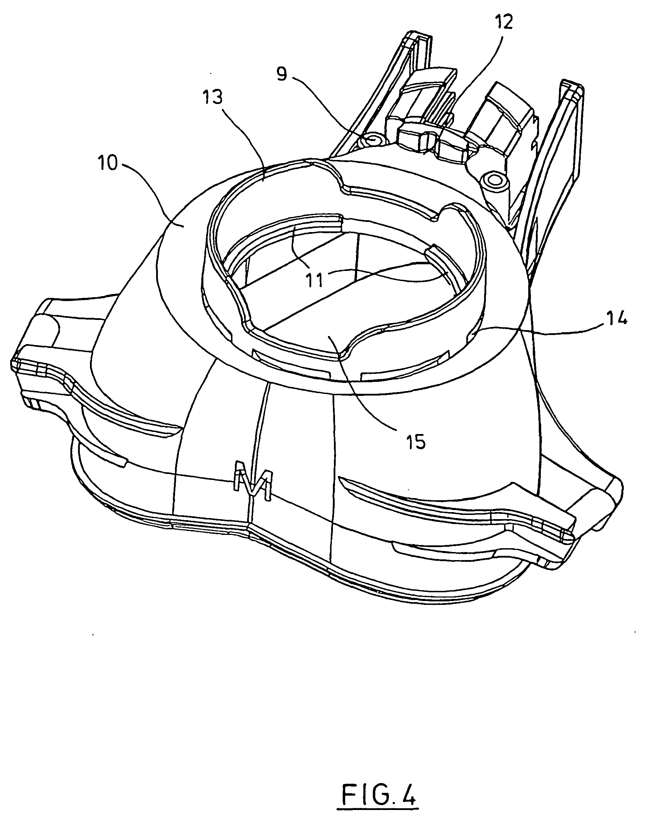

In Fig. 4 ist der Grundkörper der Nasalmaske in perspektivischer Ansicht dargestellt. Ein Zentrierring (13) ist für das Aufsetzen des Sicherungsrings (31) vorgesehen, Ausatemspalte (14) befinden sich an den Seiten des zentrierrings (13) und öffnen sich zur Ausströmfläche (10) hin. Der Zentrierring (13) weist Aussparungen (15) auf, die eine Fehlmontage des Sicherungsrings verhindern, indem sie eine mechanische Kodierung bilden.

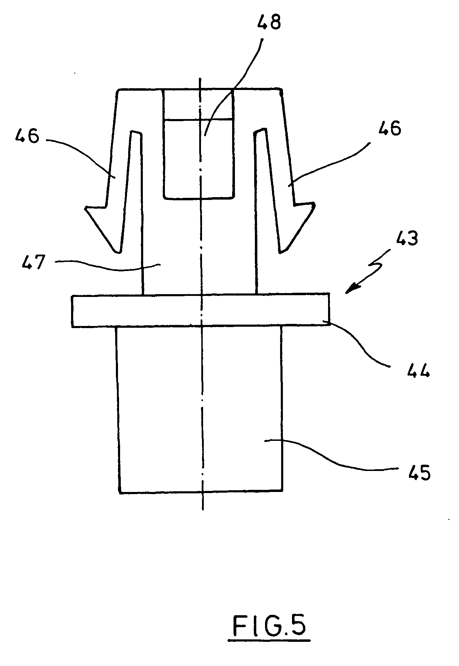

Fig. 5 zeigt ein Kupplungselement (43), das zur Verbindung von zwei nicht dargestellten Bauteilen verwendbar ist. Die Kupplung (43) weist ein Sockelelement (44) auf, das beidseitig mit mechanischen Kodierungen versehen ist. Eine der Kodierungen ist als Innenhülse (45) ausgebildet, auf die lediglich ein als Außenhülse ausgebildetes Gegenstück aufsteckbar ist. Das zweite Element weist ein mit Rastzungen (46) versehenes Zentralelement (47) auf, das mit einem Seitenprofil (48) als Kodierung versehen ist. Sowohl die Rastzungen (46) mit ihrem zugeordneten Profil und ihrer Anordnung als auch das Seitenprofil (48) stellen eigenständige Kodierungen bereit, so daß die Kupplung (43) zur vertauschungssicheren Verbindung einer Vielzahl unterschiedlicher Modulelemente verwendbar ist.

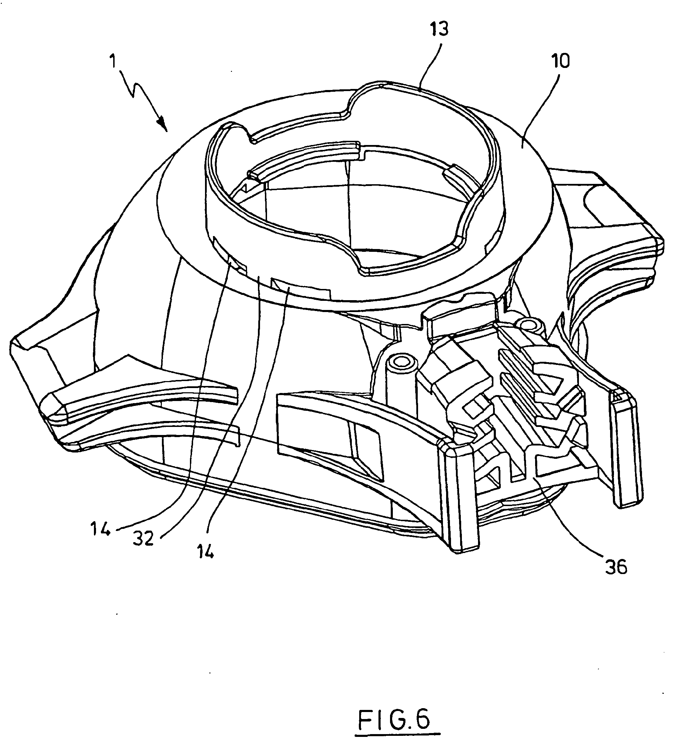

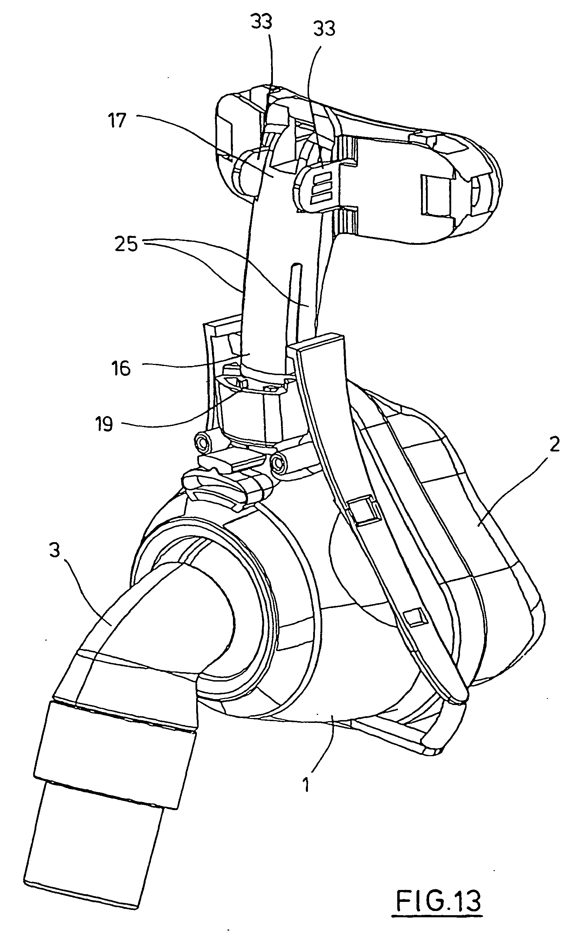

Die perspektivische Darstellung in Fig. 6 veranschaulicht nochmals den Maskenkörper (1) nach einem Abnehmen der in Fig. 13 nicht dargestellten Stirnstütze (5). Im Übergangsbereich zwischen der Ausströmfläche (10) und dem Zentrierring (13) ist insbesondere noch einmal die Anordnung der Ausatemspalte (14) zu erkennen. Die Ausatemspalte (14) besitzt im wesentlichen rechteckförmige Querschnittgestaltungen und verlaufen mit ihren Längsachsen in einer Umfangsrichtung der Ausströmfläche (10). Die einzelnen Ausatemspalte (14) sind von Distanzelementen (32) voneinander getrennt. Die Distanzelemente (32) führen zu einer mechanischen Verbindung zwischen dem Zentrierring (13) und dem weiteren Material des Maskenkörpers (1).

Die Ausatemspalte (14) sind vorzugsweise derart angeordnet, daß sie in einem der Ausströmfläche (10) zugewandten Bereich des Zentrierringes (13) verlaufen. Hierdurch wird das aus den Ausatemspalten (14) austretende Atemgas unmittelbar in den Bereich der Ausströmfläche (10) geleitet.

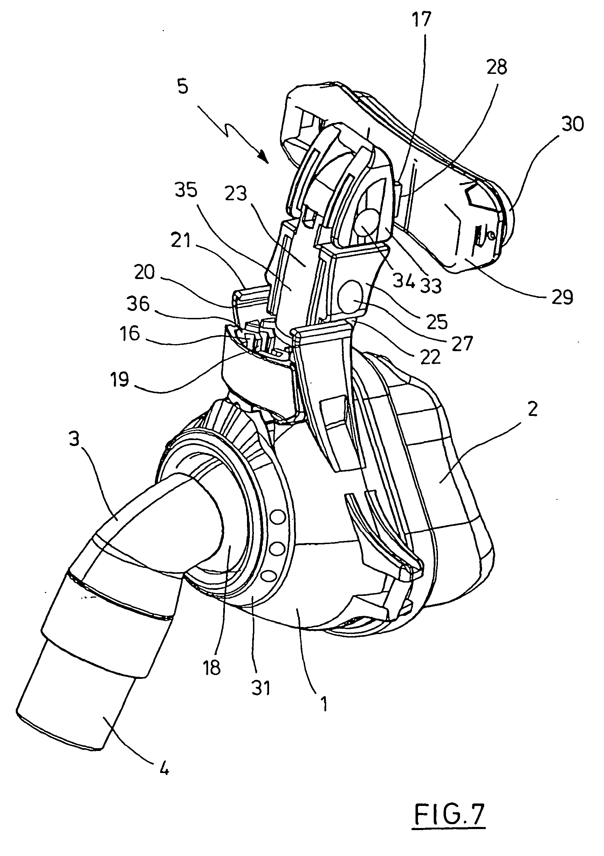

Fig. 7 zeigt eine andere Ausführungsform einer Atemmaske mit Stirnstütze (5). Zur Positionsvorgabe der Stirnstütze (5) relativ zum Maskenkörper (1) sind eine Grobverstellung (16) sowie eine Feinverstellung (17) verwendet. Derartige Verstellungen (16, 17) können auch zur Verbindung und Positionsfestlegung weiterer Bauelemente eines am Beispiel einer Atemmaske dargestellten Patienteninterfaces verwendet werden.

Im dargestellten Ausführungsbeispiel dienen die Verstellungen (16, 17) zur Positionsfestlegung der Stirnstütze (5) relativ zur Position einer nicht dargestellten Stirn eines Patienten und insbesondere zur Anpassung an unterschiedliche Verlaufskonturen einer derartigen Stirn. Die Verstellungen (16, 17) sind beim vorliegenden Ausführungsbeispiel somit im wesentlichen quer zu einer von der Maskenwulst (2) aufgespannten Bezugsebene orientiert. Beispielsweise können derartige Verstellungen (16, 17) auch zu einer Höhenfestlegung der Stirnstütze (5) oder im Zusammenhang mit anderen Anwendungen zum Einsatz kommen.

Die Grobverstellung (16) ist im Bereich der Halterung (36) angeordnet und weist mindestens zwei Halterungsausnehmungen (19) auf. In die Halterungsausnehmungen (19) ist die Stirnstütze (5) mit ihrem Schaft (35) einsteckbar und es wird hiermit gerastert eine Abstandsvorgabe zur Stirn eines Patienten realisiert. Beim dargestellten Ausführungsbeispiel werden drei Halterungsausnehmungen (19) verwendet.

Nach einem Einsetzen des Schaftes (35) in die Grobverstellung (16) erfolgt eine Arretierung unter Verwendung von Federlaschen (20), die mit einem Halterungsprofil (21) in ein entsprechendes Gegenprofil (22) des Schaftes (35) eingreifen.

Der Schaft (35) besteht aus einem Zentralsteg (23) sowie Befestigungslaschen (25) die mit Bedienmulden (27) versehen sind. Bei einem Zusammendrücken der Befestigungslaschen (25) gegeneinander werden die Gegenprofile (22) aus den Halterungsprofilen (21) der Federlaschen (20) herausgeführt und die Stirnstütze (5) kann aus der Grobverstellung (16) entnommen werden.

Die Feinverstellung (17) umfaßt einen Tragsteg (28), der eine Sockelplatte (29) der Stirnstütze haltert. An der Sokkelplatte (29) ist ein Stirnpolster (30) befestigt. Der Tragsteg (28) erstreckt sich ausgehend von der Sockelplatte (29) in eine dem Stirnpolster (30) abgewandte Richtung.

Zur Betätigung der Feinverstellung (17) sind am Schaft (35) Bedienlaschen (33) angebracht, die Bedienmulden (34) aufweisen. Bei einem Gegeneinanderdrücken der Bedienlaschen (33) wird die Feinverstellung (17) entriegelt und eine Positionsveränderung ist durchführbar.

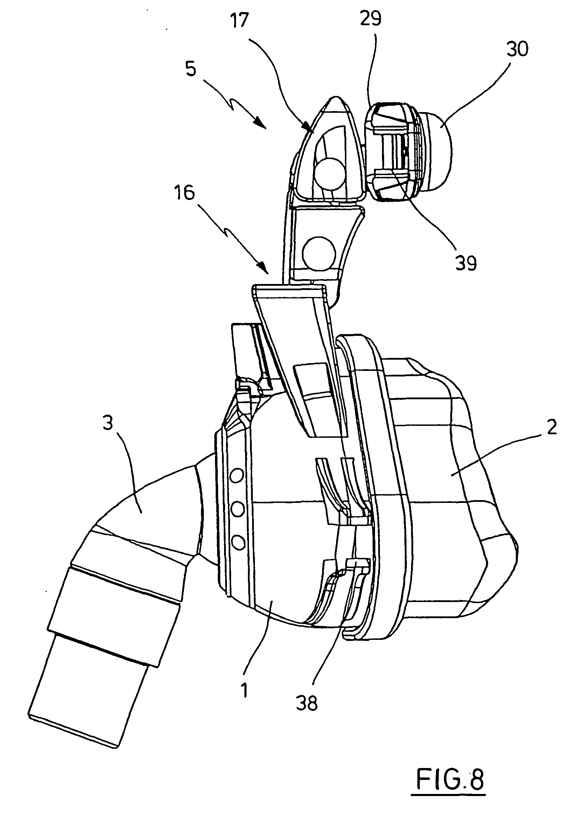

Fig. 8 zeigt die Atemmaske gemäß Fig. 7 in einer Seitenansicht. Zusätzlich zu erkennen sind Halterungselemente (38) für eine nicht dargestellte Bänderung. Weitere Halterungselemente (39) sind an der Sockelplatte (29) angeordnet, um eine Verbindung mit einer Stirnbänderung bereitzustellen.

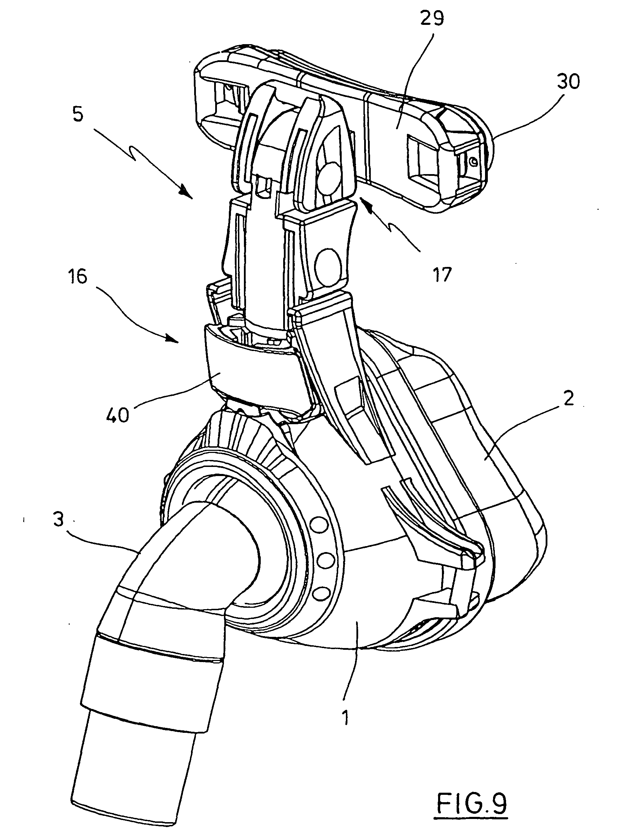

Fig. 9 veranschaulicht in einer Darstellung ähnlich wie Fig. 7 eine andere Anordnung des Schaftes (35) in der Grobverstellung (16). Ebenso wie in Fig. 7 ist die Grobverstellung (16) seitlich mit einem Abdeckelement (40) versehen.

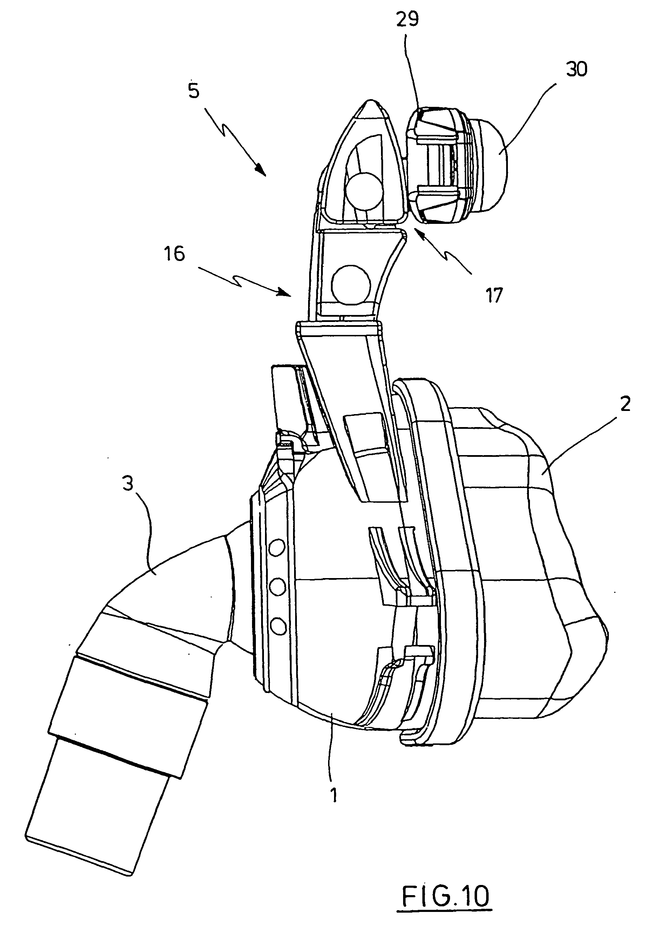

Fig. 10 veranschaulicht die Anordnung gemäß Fig. 9 in einer Seitenansicht. Gegenüber der Darstellung in Fig. 8 wurde lediglich die Positionierung der Grobverstellung (16) verändert, die Positionierung der Feinverstellung (17) wurde beibehalten.

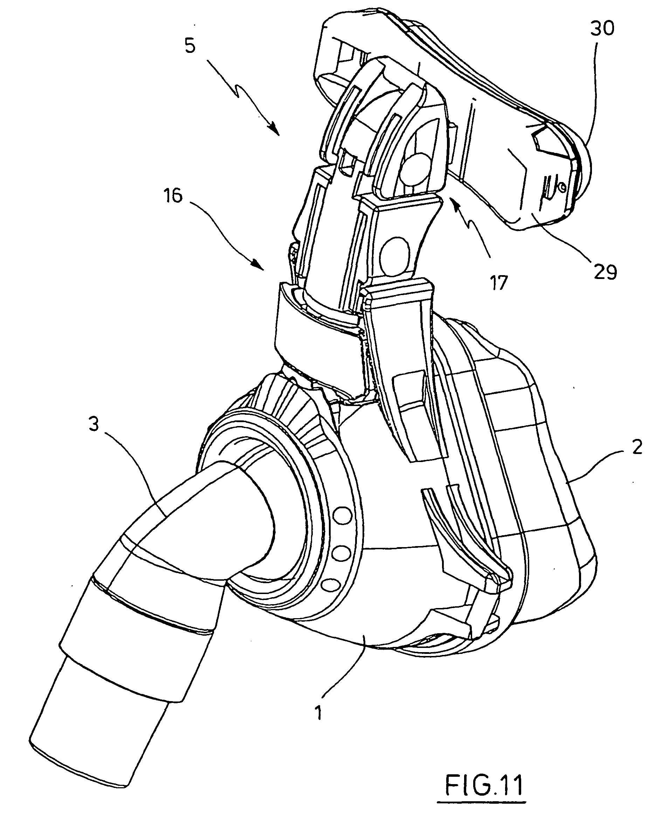

Fig. 11 zeigt die Anordnung gemäß Fig. 9 in einer nochmals geänderten Positionierung, bei der die Stirnstütze (5) einen weiteren Schritt von der Stirn eines nicht dargestellten Patienten entfernt wurde. Bei der gemäß dem Ausführungsbeispiel vorgesehenen dreistufigen Grobverstellung (16) wird hierdurch die größte Abstandsvorgabe zum Patienten realisiert.

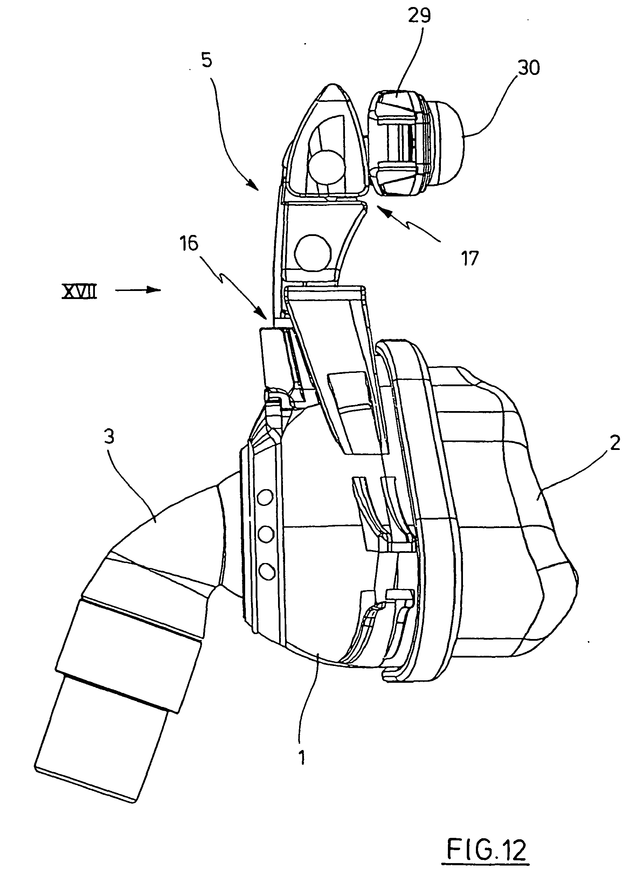

Die Anordnung gemäß Fig. 12 zeigt die Positionierung gemäß Fig. 11 in einer Seitenansicht. Gegenüber der Anordnung in Fig. 10 wurde wiederum lediglich die Positionierung der Grobverstellung (16) geändert, die Feinverstellung (17) wurde unverändert beibehalten.

Fig. 13 zeigt eine abgewandelte Ausbildung der Grobverstellung (16) und der Feinverstellung (17). Die Geometrien der einzelnen Bauteile sind hierbei gegenüber den vorstehend erläuterten Ausführungsformen vereinfacht, es liegt dafür aber ein weniger geschlossener Außenkonturverlauf vor.

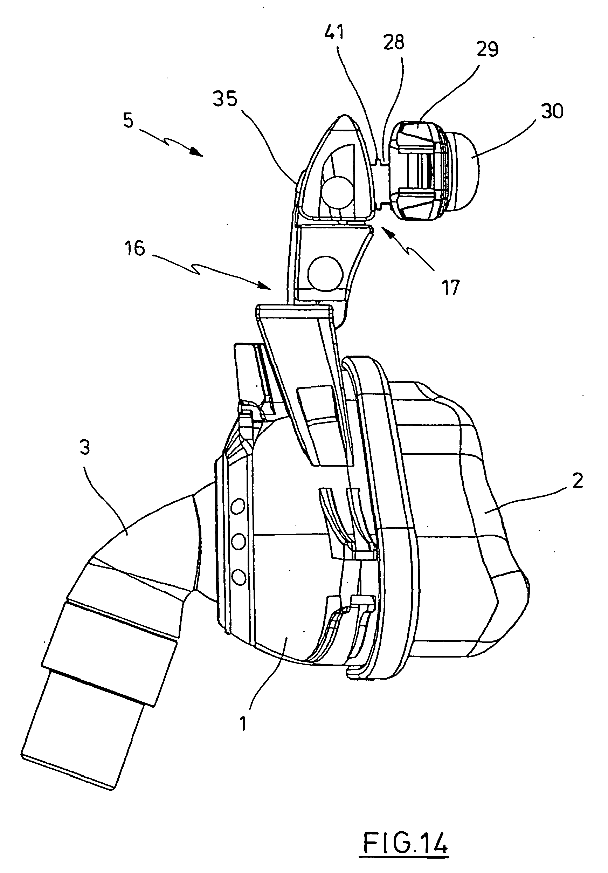

Fig. 14 zeigt die Atemmaske gemäß Fig. 14 mit unveränderter Positionierung der Grobverstellung (16) und veränderter Positionierung der Feinverstellung (17). Der Tragsteg (28) wurde hierbei um eine Rastpositionierung aus dem Schaft (35) herausgezogen. Zu erkennen ist insbesondere ein am Tragsteg (28) angeformtes Profil (41), das als Rastung für die Feinverstellung (17) dient.

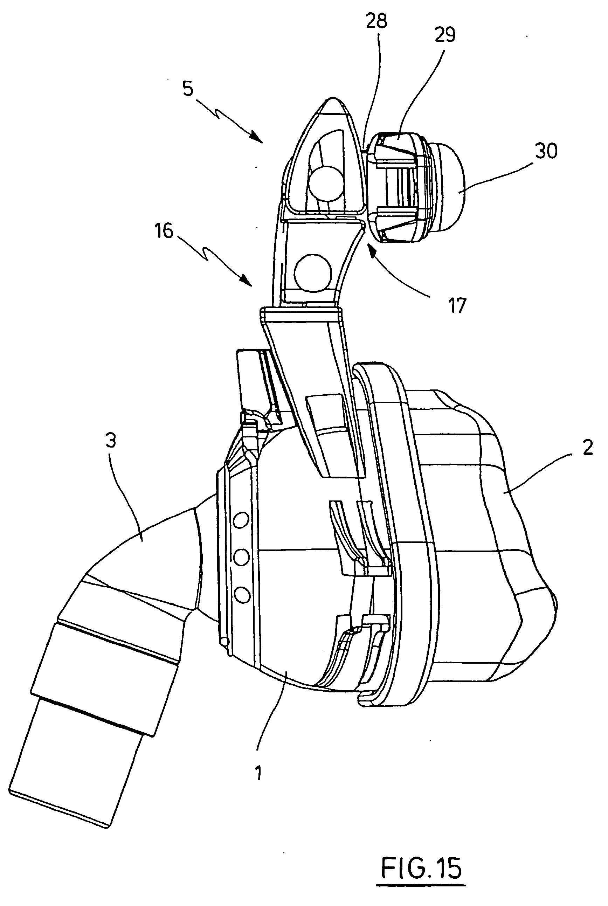

Gemäß dem in Fig. 15 dargestellten Betriebszustand wurde die Positionierung der Stirnstütze (5) gegenüber der Anordnung in Fig. 14 derart verändert, daß die Sockelplatte (29) mit ihrem Tragsteg (28) um 180° Grad verdreht in die Feinverstellung (17) eingeschoben wurde. Aufgrund einer bezüglich einer Höhenausdehnung der Sockelplatte (29) unsymmetrischen Anordnung des Tragsteges (28) kann hierdurch eine Höhenveränderung des Stirnpolsters (30) hervorgerufen werden.

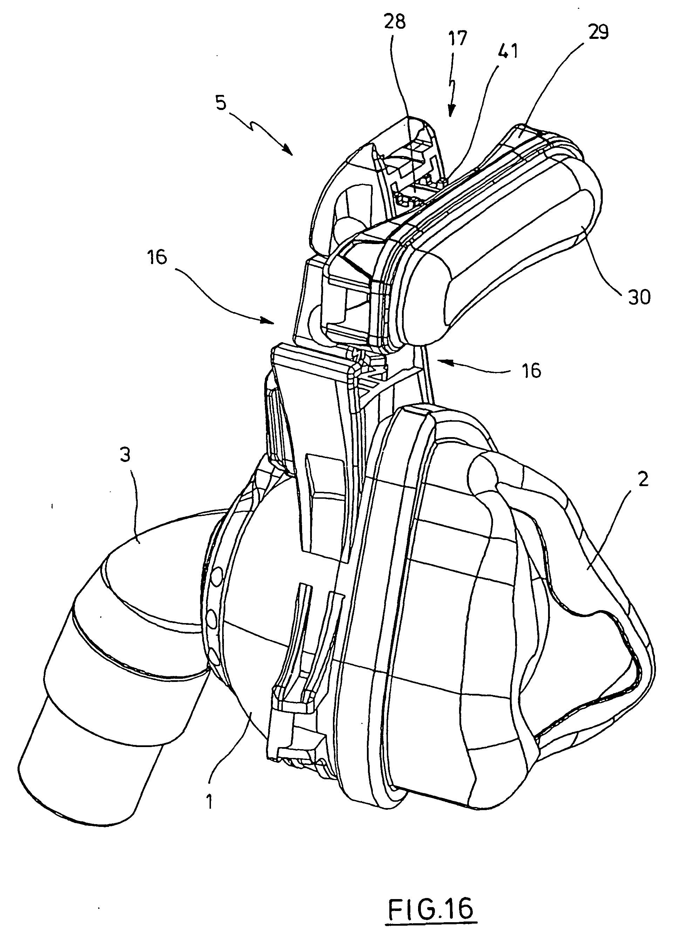

Fig. 16 zeigt eine weitere perspektivische Darstellung der Anordnung gemäß Fig. 15. Es ist insbesondere zu erkennen, daß in der Längsrichtung des Tragsteges (28) eine Mehrzahl von Vorsprüngen zur Ausbildung eines gerasterten Profils (41) angeordnet sind.

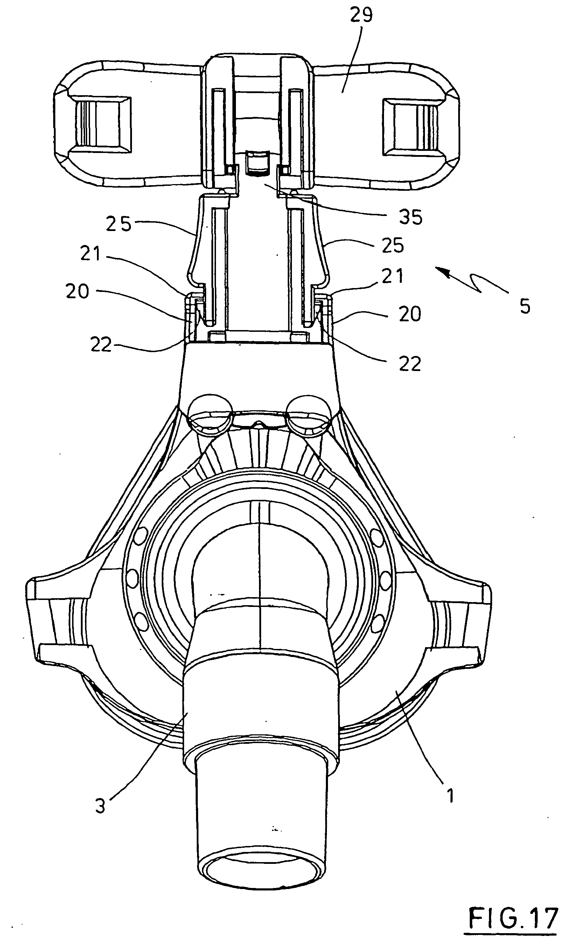

Fig. 17 zeigt eine Seitenansicht der Atemmaske in Fig. 12 gemäß Blickrichtung XVII. Zu erkennen ist insbesondere das Ineinandergreifen des Halterungsprofils (21) der mit dem Maskenkörper (1) verbundenen Federlaschen (20) sowie der am Schaft (35) angeordneten Befestigungslaschen (25) mit ihren Gegenprofilen (22).

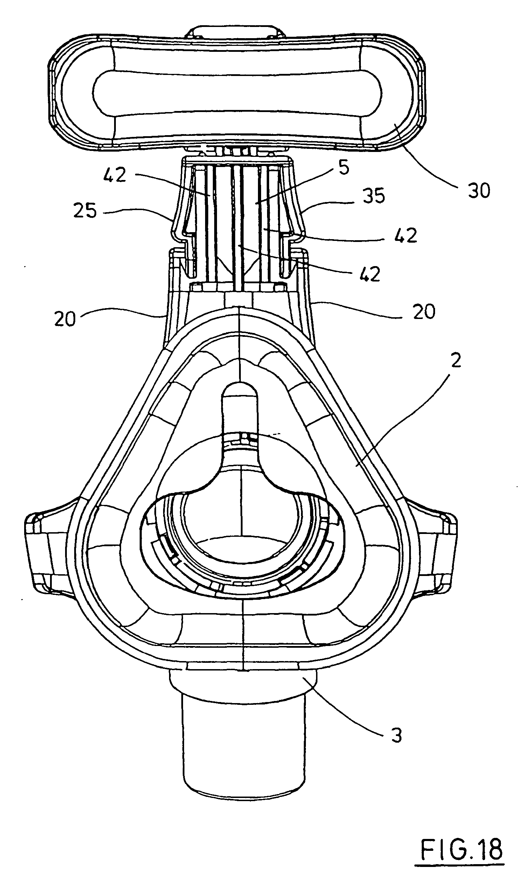

Fig. 18 zeigt die Anordnung gemäß Fig. 17 bei einer Blickrichtung von hinten. Zu erkennen ist hierbei insbesondere, daß der Schaft (35) wenigstens bereichsweise als ein Profilelement ausgebildet ist, das Verstrebungen (42) aufweist. Hierdurch kann bei einem geringen Baugewicht und einem geringen Materialeinsatz eine hohe Stabilität erreicht werden. Darüber hinaus zeigt Fig. 18 ähnlich wie Fig. 17 das Eingreifen der Befestigungslaschen (25) in die Federlaschen (20).

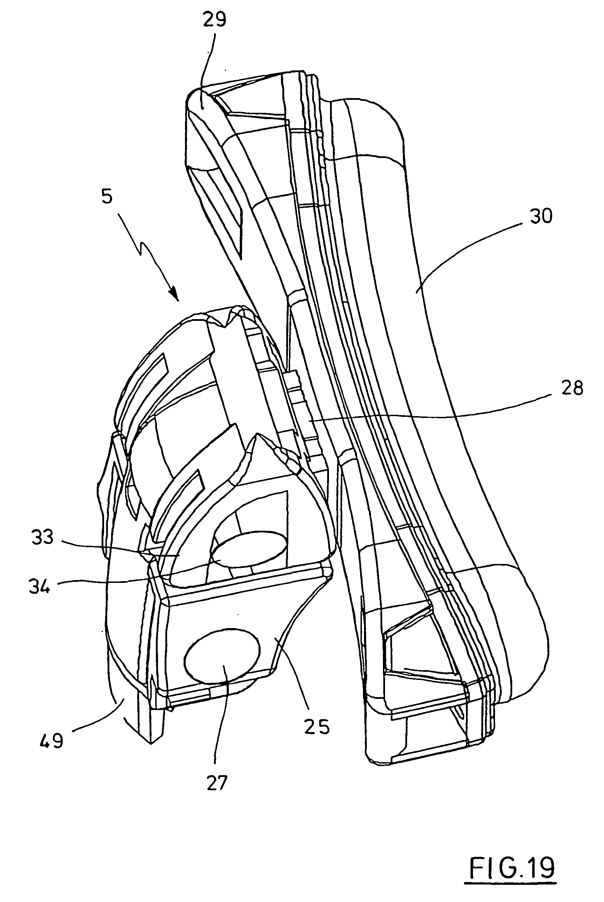

Fig. 19 zeigt die Stirnstütze (5) in einer weiteren perspektivischen Darstellung, um insbesondere noch einmal die Anordnung der Befestigungslaschen (25) sowie der Bedienlaschen (33) zu veranschaulichen. Gut zu erkennen ist bei dieser Darstellung ein als Teil der in Fig. 7 dargestellten Grobverstellung (16) ausgebildeter Sockelsteg des Schaftes (35), der zur Einführung in eine der ebenfalls in Fig. 7 dargestellten Halterungsausnehmungen (19) vorgesehen ist.

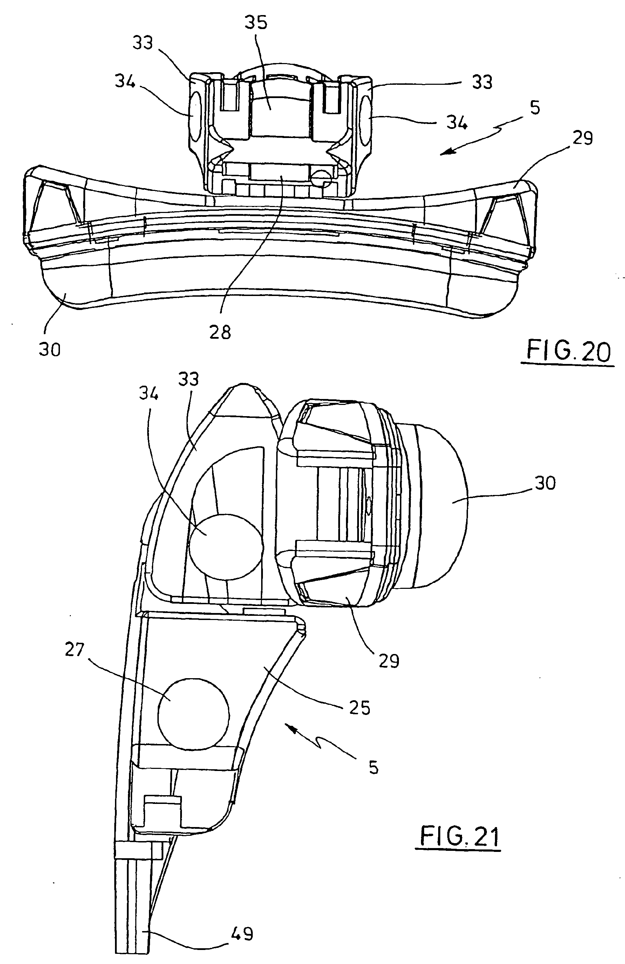

Die Draufsicht auf die Stirnstütze (5) gemäß Fig. 19 in Fig. 20 veranschaulicht insbesondere noch einmal die Anordnung der Bedienlaschen (33) am Schaft (35). Die Sockelplatte (29) ist bei dieser Anordnung mit dem Tragsteg (28) möglichst weit in den Schaft (35) hineingeschoben.

Aus der Seitenansicht in Fig. 21 ist ebenfalls noch einmal die Anordnung der Befestigungslaschen (25), der Bedienlaschen (33) und des Sockelsteges (49) relativ zur Sockelplatte (29) und zum Stirnpolster (30) zu erkennen.

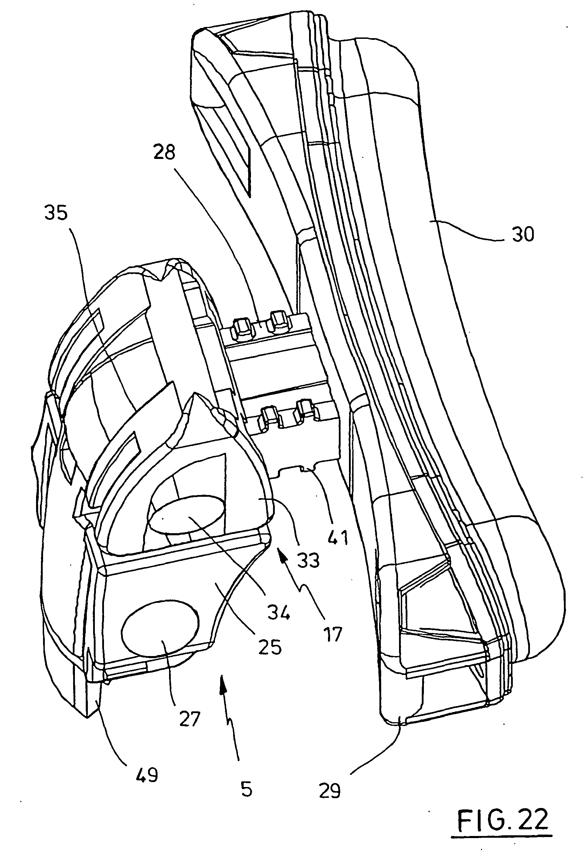

Fig. 22 zeigt eine Anordnung ähnlich zu Fig. 19, jedoch mit einer veränderten Positionierung im Bereich der Feinverstellung. Die Sockelplatte (29) ist hier mit dem Tragsteg (28) weiter aus dem Schaft (35) herausgezogen. Durch das Profil (41) erfolgt eine Arretierung der gewählten Positionierung im Bereich der Feinverstellung (17).

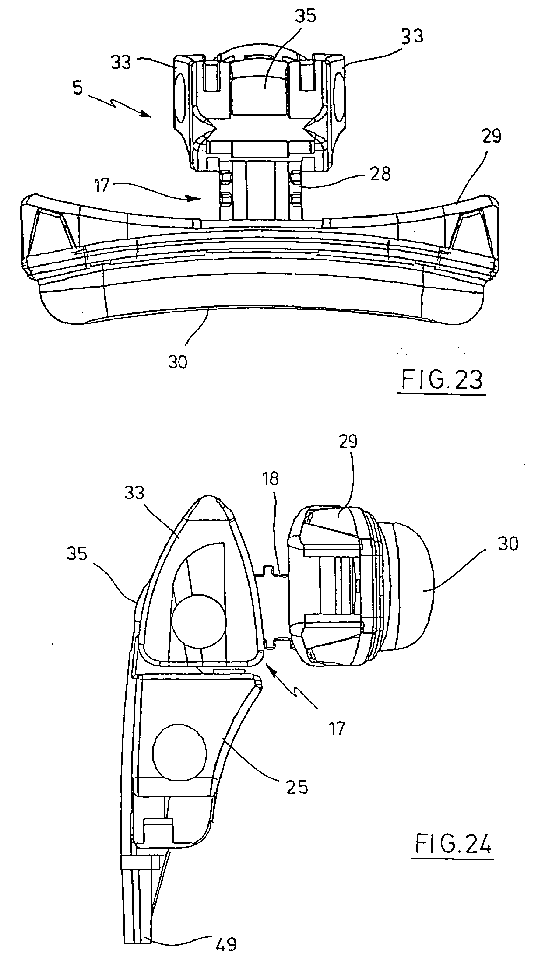

Fig. 23 zeigt eine Draufsicht auf die Anordnung von Fig. 22 und veranschaulicht insbesondere die Halterung des Tragsteges (28) unter Verwendung der Feinverstellung (17) im Bereich des Schaftes (35). Die Seitenansicht in Fig. 24 veranschaulicht ebenfalls nochmals die in Fig. 22 perspektivisch dargestellte Anordnung mit aus dem Schaft (35) teilweise herausgezogenem und von der Feinverstellung (17) gehalterten Tragsteg (28).

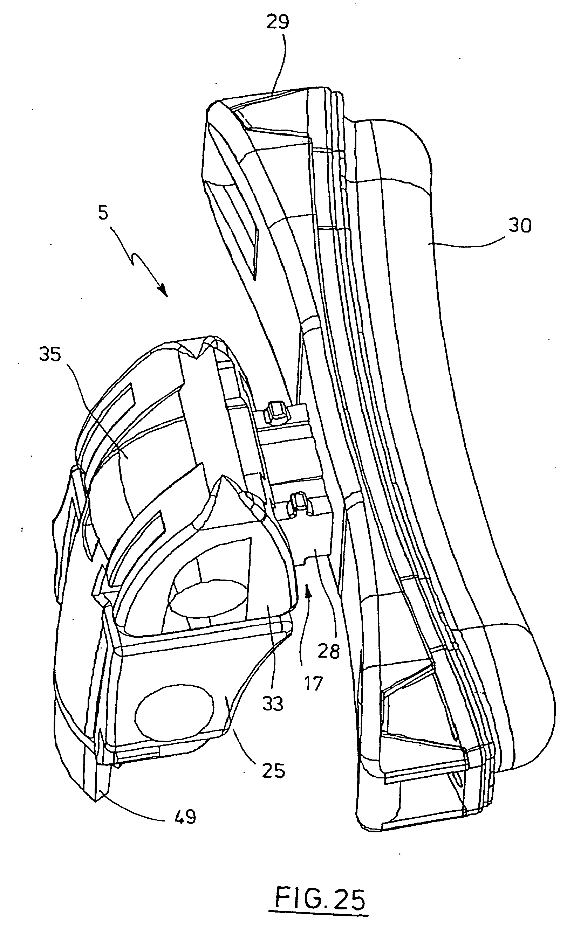

Fig. 25 entspricht im wesentlichen der Darstellung in Fig. 19 und Fig. 22, es wird allerdings eine zwischen den Anordnungen in Fig. 19 und Fig. 22 liegende Positionierung der Sockelplatte (29) relativ zum Schaft (35) unter Verwendung der Feinverstellung (17) gezeigt.

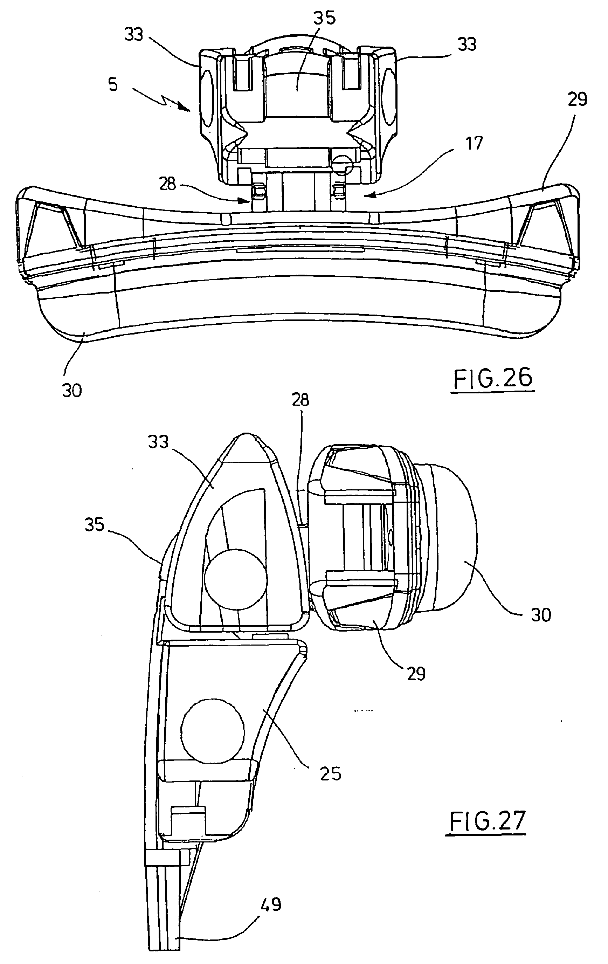

Ähnlich wie Fig. 23 und Fig. 24 in Relation zu Fig. 22 zeigen Fig. 26 und Fig. 27 eine Draufsicht bzw. eine Seitenansicht für die Positionierung der Feinverstellung (17) entsprechend Fig. 25.

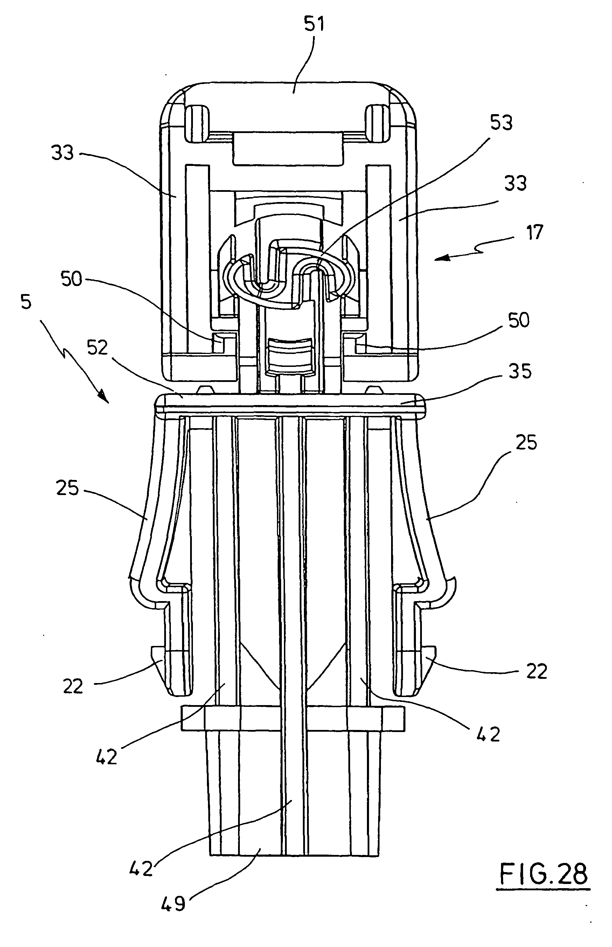

Fig. 28 zeigt eine vergrößerte Darstellung des Schaftes (35) der Stirnstütze (5) bei einer Blickrichtung entsprechend Fig. 18 und vom Schaft (35) abgenommener Sockelplatte (29). Fig. 28 veranschaulicht in großer Detailliertheit die Anordnung der Bedienlaschen (33) für die Feinverstellung (17). Die Bedienlaschen (33) sind mit einem Profil (50) für einen Eingriff in das in Fig. 22 dargestellte Profil (41) des Tragsteges (28) versehen. Bei einem zusammendrücken der Bedienlaschen (33) werden die Profile (41, 50) voneinander getrennt und eine Verstellung des Tragsteges (28) ist möglich. Die Bedienlaschen (33) sind im Bereich ihrer den Profilen (50) abgewandten Ausdehnung von einer Brücke (51) miteinander verbunden. Eine Verbindung der Befestigungslaschen (25) erfolgt im Bereich einer den Gegenprofilen (22) abgewandten Ausdehnung durch einen Quersteg (52).

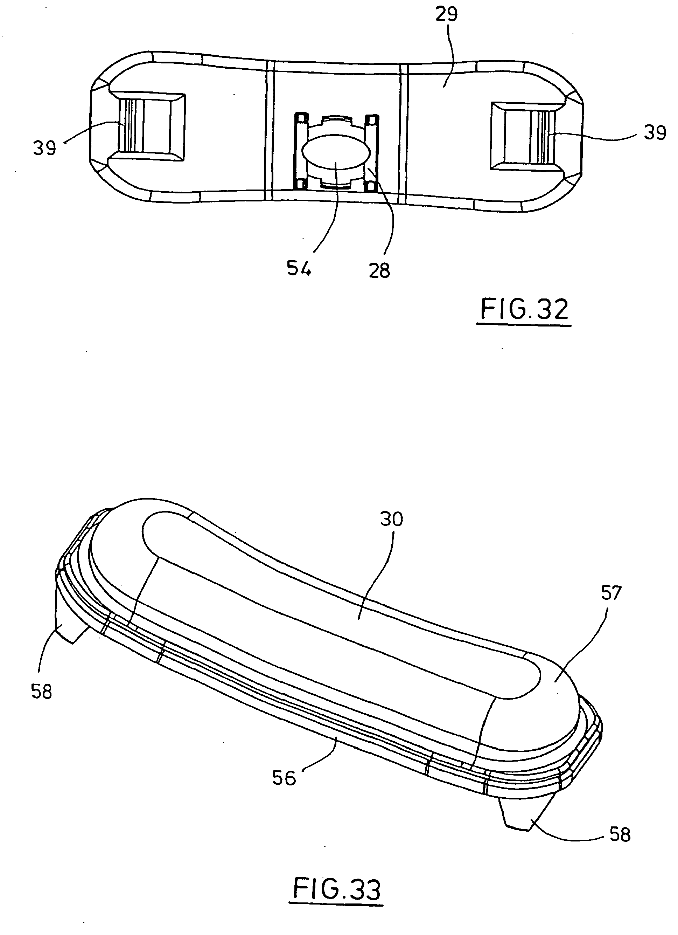

Zur Führung des in Fig. 22 dargestellten Tragsteges (28) ist der Schaft (35) mit einem Tragprofil (53) versehen, das beim dargestellten Ausführungsbeispiel die Gestalt eines liegenden "s" aufweist. Das Tragprofil (53) greift in eine Führungsausnehmung des Tragsteges (28) ein, die beispielsweise in Fig. 32 dargestellt ist. Die konstruktive Realisierung des Tragprofils (53) stellt eine hohe Stabilität bei geringem Materialeinsatz bereit. Trotz der relativ groß dimensionierten Führungsausnehmung (54) kann das Tragprofil (53) durch die gewählte Gestaltung mit einer vergleichsweise geringen Wandstärke bei guter Entformbarkeit hergestellt werden.

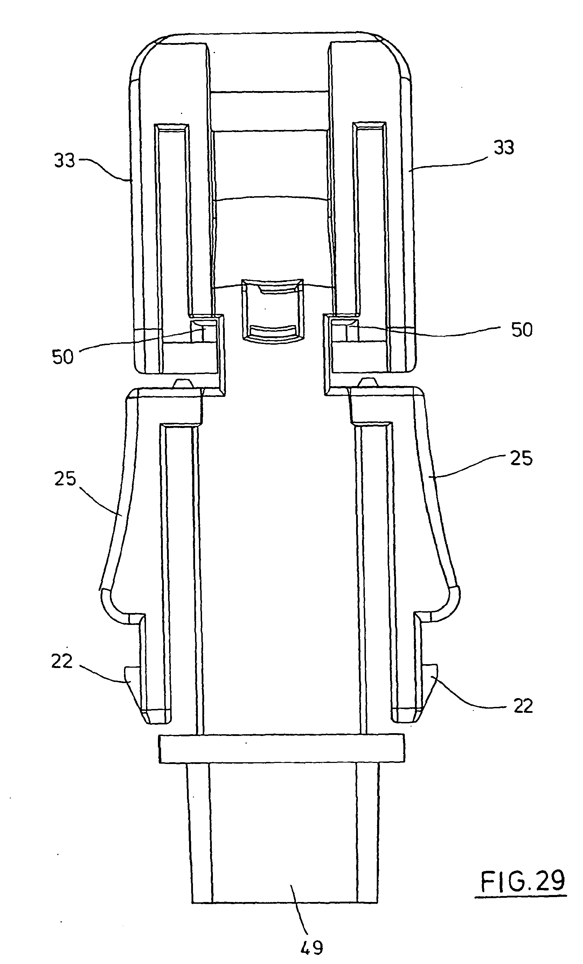

Fig. 29 zeigt den Schaft (35) gemäß Fig. 28 bei einer Blickrichtung von hinten. Auch bei dieser Ansicht ist insbesondere zu erkennen, daß durch die gewählte konstruktive Realisierung des Schaftes (35) eine hohe mechanische Stabilität bei vergleichsweise dünnen und gleichmäßigen Wandstärken erreicht wird. Diese konstruktive Realisierung unterstützt insbesondere eine Herstellung aus Kunststoff im Spritzgußverfahren.

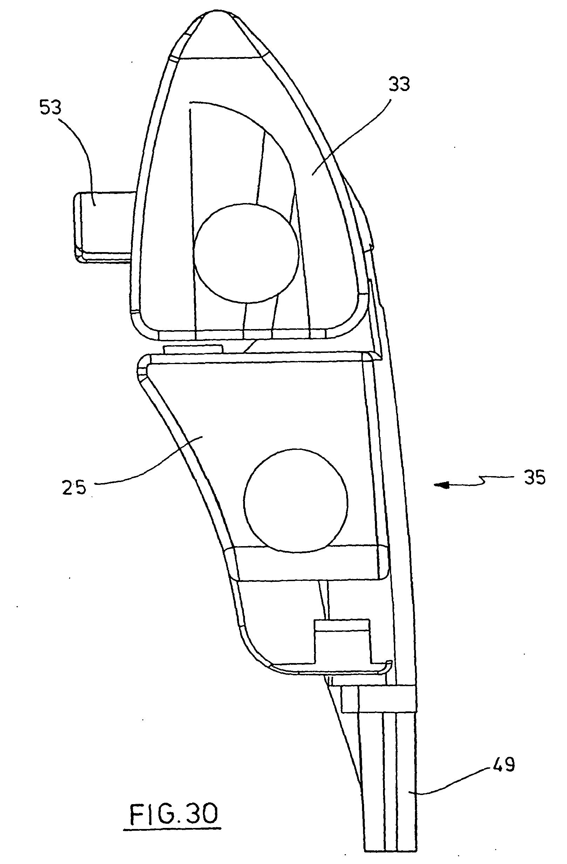

Die Seitenansicht in Fig. 30 veranschaulicht insbesondere, daß das Tragprofil (53) relativ weit aus dem Schaft (35) hervorsteht und hierdurch eine gute Abstützung des in Fig. 22 gezeigten Tragsteges (28) entlang eines langen Verstellweges gewährleistet.

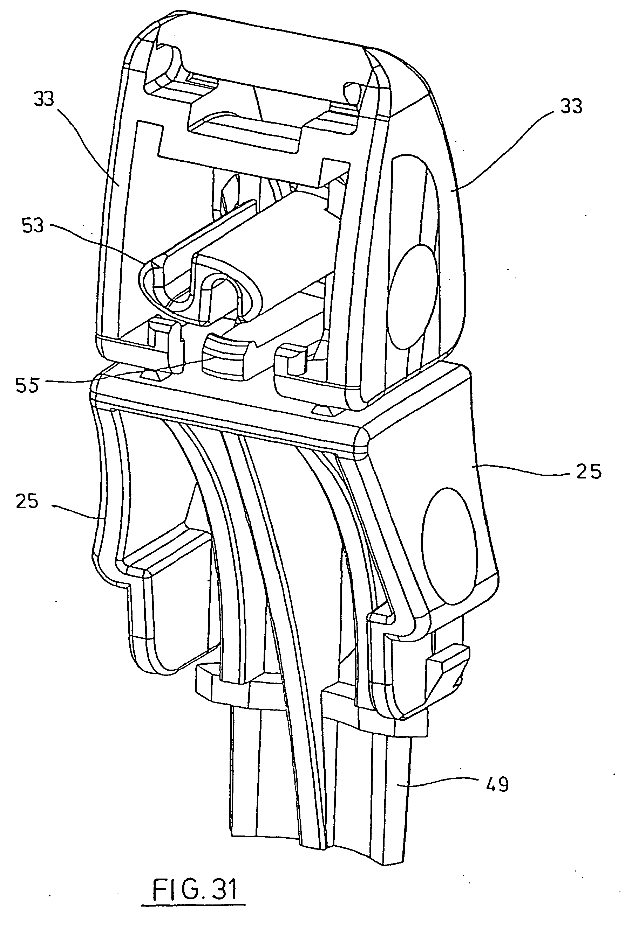

Die Darstellung in Fig. 31 veranschaulicht die relativ lange räumliche Dimensionierung des Tragprofils (53). Fig. 31 zeigt ebenfalls die Anordnung eines Anschlages (55), der zu einer Begrenzung des Verstellweges des Tragsteges (28) innerhalb der Feinverstellung (17) dient.

Fig. 32 zeigt eine rückwärtige Ansicht der Sockelplatte (29) mit Tragsteg (28). Zu erkennen ist insbesondere die Führungsausnehmung (54) des Tragsteges (28). Ebenfalls sind die Halterungselemente (39) zur Befestigung einer Stirnbänderung zu erkennen. Fig. 33 zeigt das auf die Sockelplatte (29) aufsteckbare Stirnpolster (30). Das Stirnpolster (30) besteht aus einem Rahmen (56) und einem Anlageelemente (57). Das Anlageelement (57) ist aus einem weichen und vorzugsweisen elastomeren Material ausgebildet, der Rahmen (56) besteht aus einem härteren Material und weist Halterungsvorsprünge (58) auf.

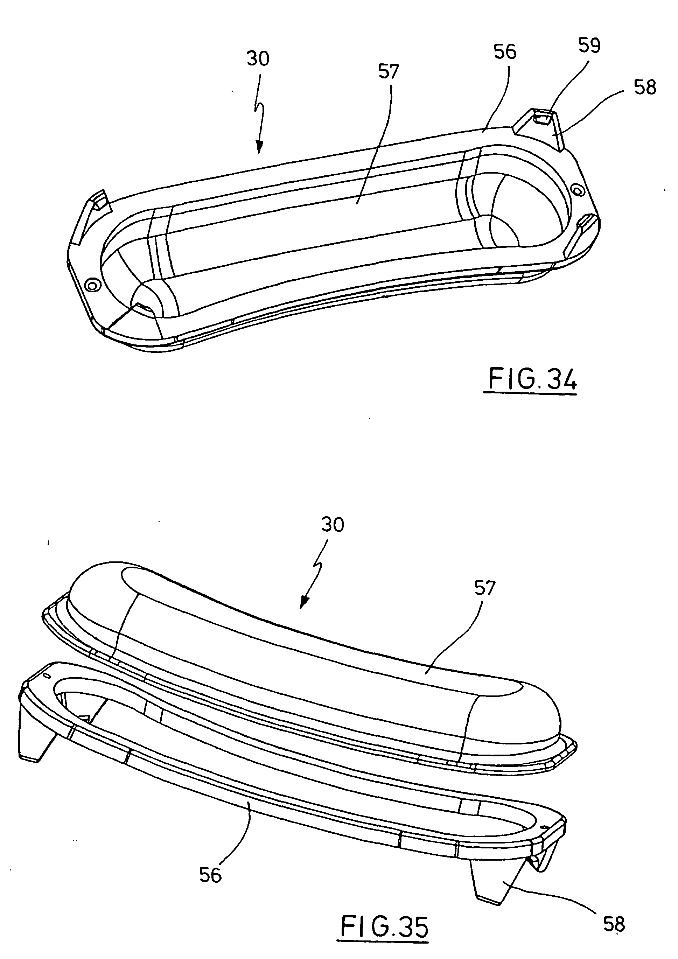

Aus der Darstellung in Fig. 34 ist erkennbar, daß die Halterungsvorsprünge (58) aus einer vom Rahmen (56) aufgespannten Ebene herausstehen und Halterungsprofile (59) besitzen. Nach einem Aufstecken des Rahmens (56) verrasten die Halterungsprofile (59) in der Sockelplatte (29).

Die Explosionsdarstellung in Fig. 35 zeigt eine voneinander getrennte Anordnung des Rahmens (56) und des Anlageelementes (57). Bei einer üblichen Verwendung ist eine derartige Trennung aber nicht möglich, da der Rahmen (56) und das Anlageelement (57) miteinander verklebt sind oder einteilig in einer Zweikomponententechnik hergestellt wurden.

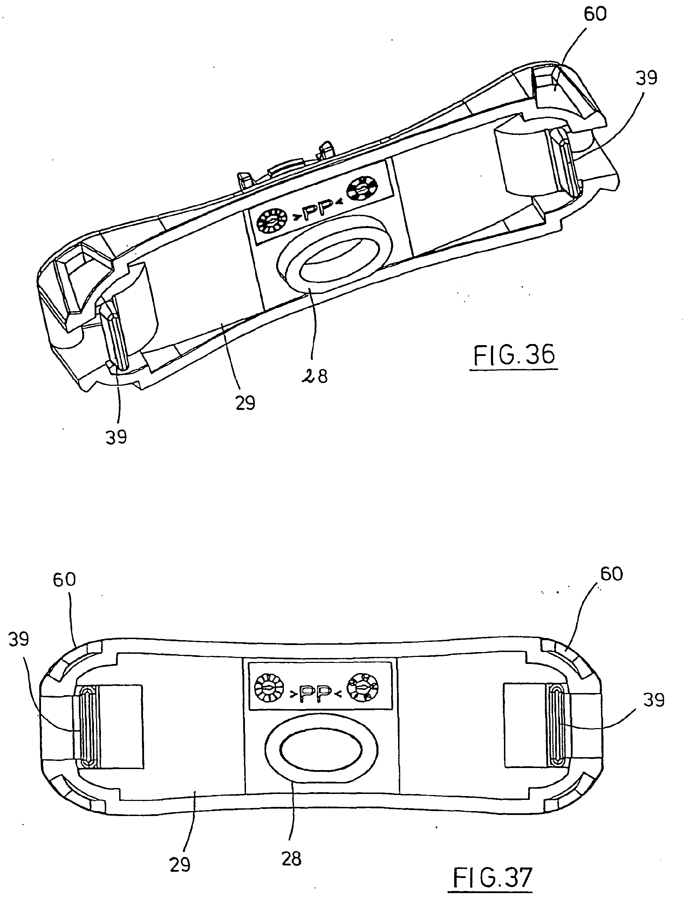

Aus der perspektivischen Darstellung der Sockelplatte (29) in Fig. 36 ist zu erkennen, daß die Sockelplatte (29) Führungsausnehmungen (60) zur Aufnahme und Arretierung der in Fig. 35 dargestellten Halterungsvorsprünge (58) aufweist. Die Konturen der Führungsausnehmungen (60) und der Halterungsvorsprünge (58) sind aneinander angepaßt, so daß in einer Querrichtung ein Formschluß realisiert wird.

Fig. 37 veranschaulicht insbesondere die sehr symmetrische Gestaltung der Sockelplatte (29), die zu einer hohen Festigkeit und einer gleichmäßigen Materialverteilung beiträgt.

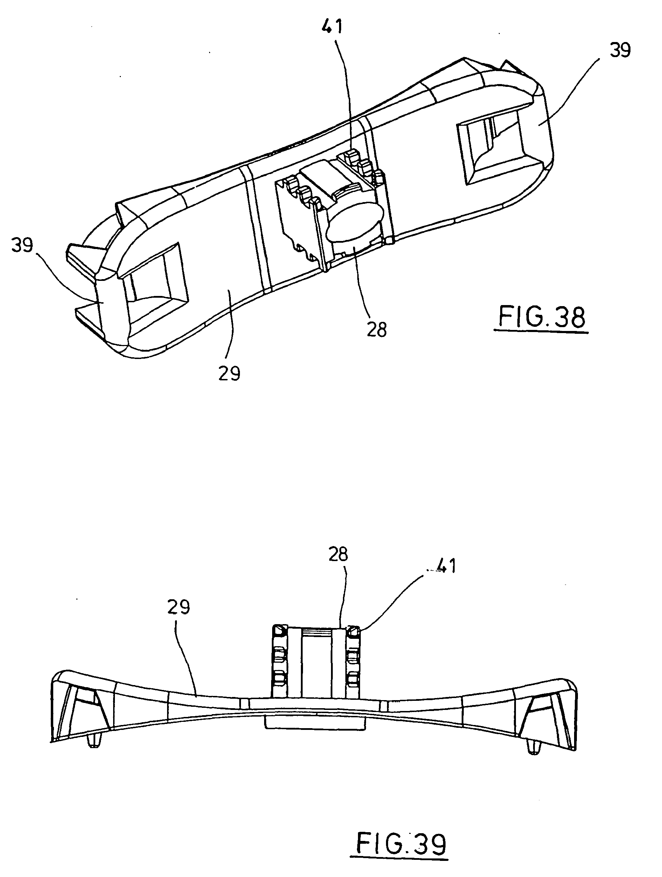

Die perspektivische Darstellung in Fig. 38 zeigt die Sokkelplatte (29) gemäß Fig. 36 bei einer Blickrichtung von hinten. Fig. 38 sowie die in Fig. 39 dargestellte Draufsicht veranschaulichen insbesondere noch einmal die Anordnung des verzahnungsartigen Profils (41) am Tragsteg (28), um gemeinsam mit den in Fig. 22 dargestellten Bedienlaschen (33) die Feinverstellung (17) bereitzustellen.

Eine weitere erfindungsgemäße Ausführungsform ist darin zu sehen, daß die Grobverstellung (16) einen im Bereich von Halterungsausnehmungen (19) des Maskenkörpers (1) einsteckbaren Schaft (35) der Stirnstütze (5) sowie eine durch federnde Befestigungslaschen (25) bereitgestellte Arretierung umfaßt.

Dabei ist auch daran gedacht, den Schaft (35) und die Halterungsausnehmungen (19) derart zu gestalten, daß der Schaft (35) innerhalb der Halterungsausnehmungen (19) schwenkbar beweglich ist und fixiert werden kann. Die Bewegungsrichtung ist dabei bevorzugt zum Anwender hin orientiert. Durch die Bewegung des Schaftes in der Halterungsausnehmung (19), beispielsweise zum Anwender hin, wird die Position der Stirnstütze (5) relativ zur Stirn eines Anwenders in zwei Dimensionen verändert. Somit sind gleichzeitig beispielsweise die Höhe der Stirnstütze (5) und die Neigung der Stirnstütze (25) veränderbar.

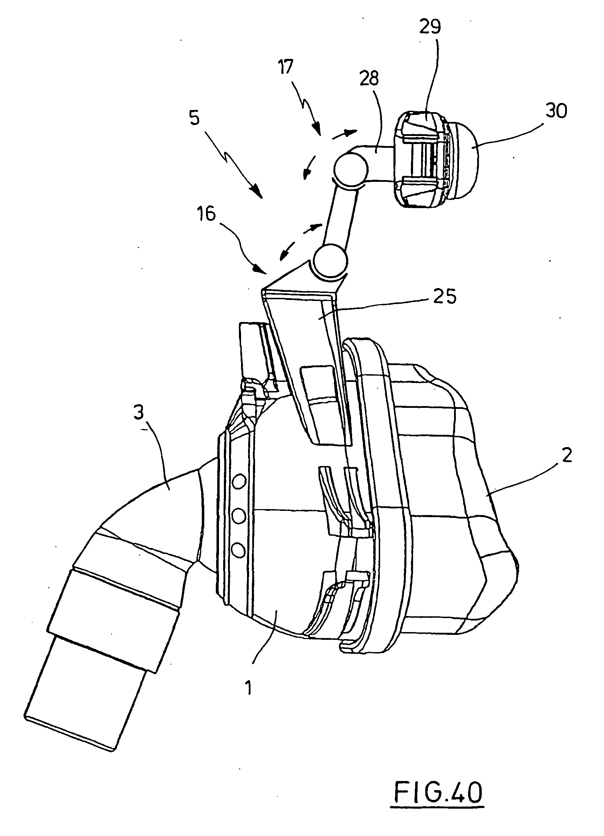

Eine weitere Ausführungsvariante besteht gemäß Fig. 40 darin, daß die Feinverstellung (17) schwenkbar beweglich ist und fixiert werden kann. Die Bewegungsrichtung ist dabei bevorzugt zum Anwender hin orientiert. Durch die Bewegung im Bereich der Feinverstellung (17) wird die Position des Stirnpolsters (30) relativ zur Stirn eines Anwenders in zwei Dimensionen verändert. Somit sind gleichzeitig beispielsweise die Höhe des Stirnpolsters (30) und die Neigung des Stirnpolsters (30) veränderbar.

Dadurch ist die Feinverstellung (17) schwenkbar, also mit einer Bewegung in zwei Dimensionen veränderbar, und die Grobverstellung (16) mit einem im Bereich von Halterungsausnehmungen (19) des Maskenkörpers (1) umsteckbaren Schaft (5) zu realisieren.

Alternativ kann außerhalb der Erfindung die Grobverstellung (16) schwenkbar sein, also mit einer Bewegung in zwei Dimensionen veränderbar, und die Feinverstellung (17) mit einem im Bereich von Halterungsausnehmungen (19) des Maskenkörpers (1) umsteckbaren Schaft (5) zu realisieren.

Außerhalb der Erfindung ist weiterhin daran gedacht, die Grobverstellung (16) und die Feinverstellung (17) schwenkbar, also jeweils mit einer Bewegung in zwei Dimensionen veränderbar zu realisieren.

Beispielsweise ist daran gedacht die Schwenkbarkeit durch ein Kugelgelenk zu realisieren.

Gemäß des in Fig. 40 dargestellten Beispiel endet der Tragsteg (28) der Stirnstütze (5) im Bereich eines ersten Schwenkgelenkes (61), das über ein Distanzstück (62) mit einem zweiten Schwenkgelenk (63) verbunden ist. Durch eine Überlagerung der Drehbewegungen der Schwenkgelenke (61, 63) und die beabstandete Anordnung der Schwenkgelenke (61, 63) durch das Distanzstück (62) kann sowohl eine Höhenjustierung als auch eine Abstandsjustierung der Stirnstütze (5) durchgeführt werden. Sowohl die Grobverstellung (16) als auch die Feinverstellung (17) sind somit bei dieser Ausführungsform durch Schwenkgelenke realisiert.

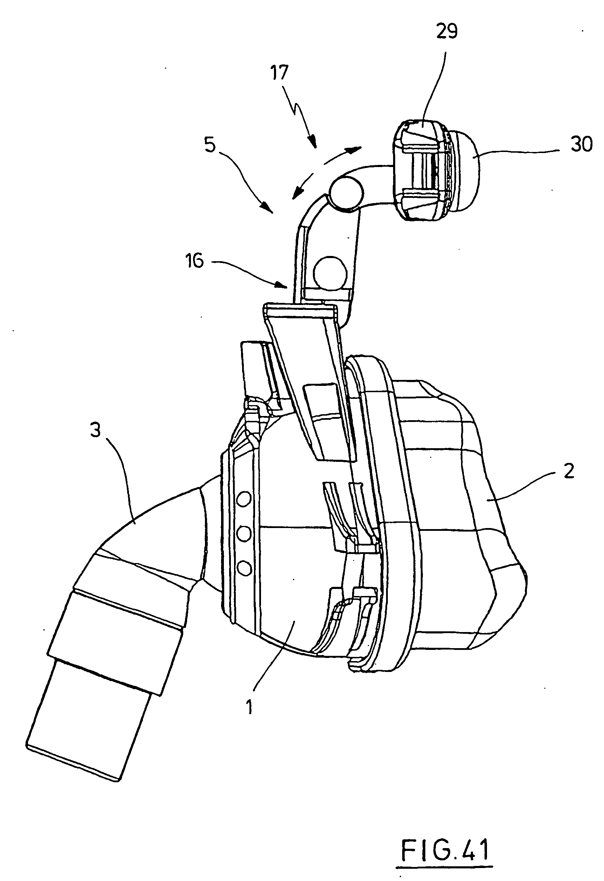

Gemäß dem Beispiel in Fig. 41 ist die Grobverstellung (16) im Bereich des Maskengrundkörpers (1) umsteckbar ausgebildet und lediglich die Feinverstellung (17) ist durch ein Schwenkgelenk realisiert.

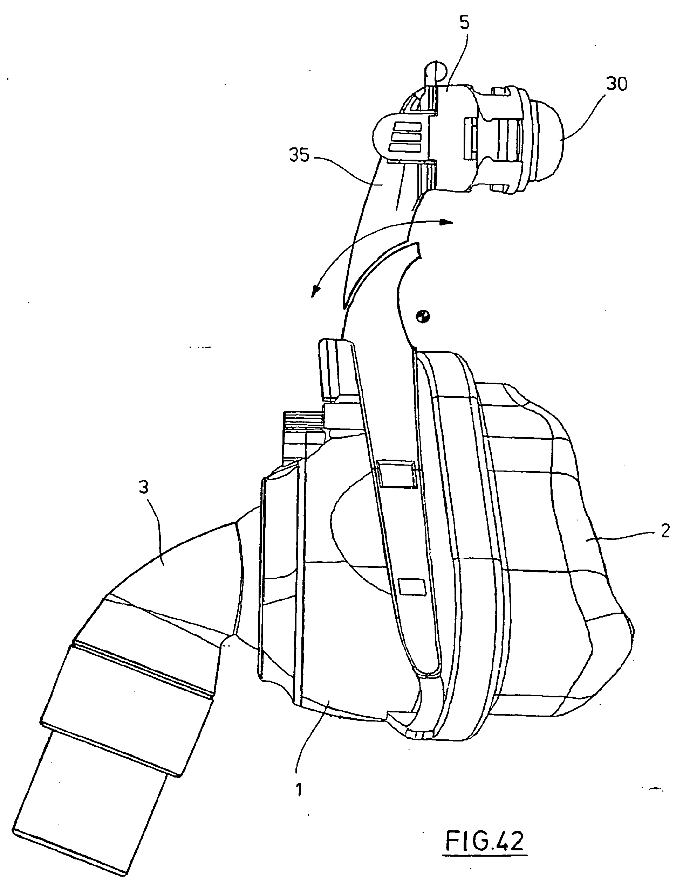

Gemäß dem Beispiel in Fig. 42 werden zur Durchführung einer Positionierung der Stirnstütze (5) gewölbte Führungsflächen (64, 65) verwendet, entlang derer der Schaft (35) relativ zum Grundkörper (1) positionierbar ist.

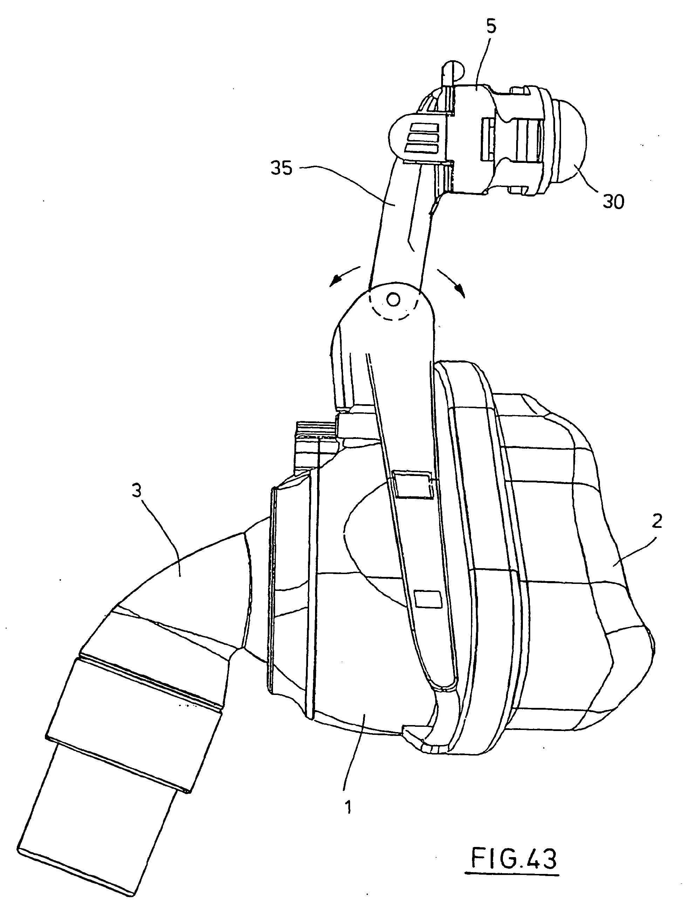

Fig. 43 zeigt einem Beispiel bei dem der Schaft (35) verschwenkbar mit dem Grundkörper (1) verbunden ist.

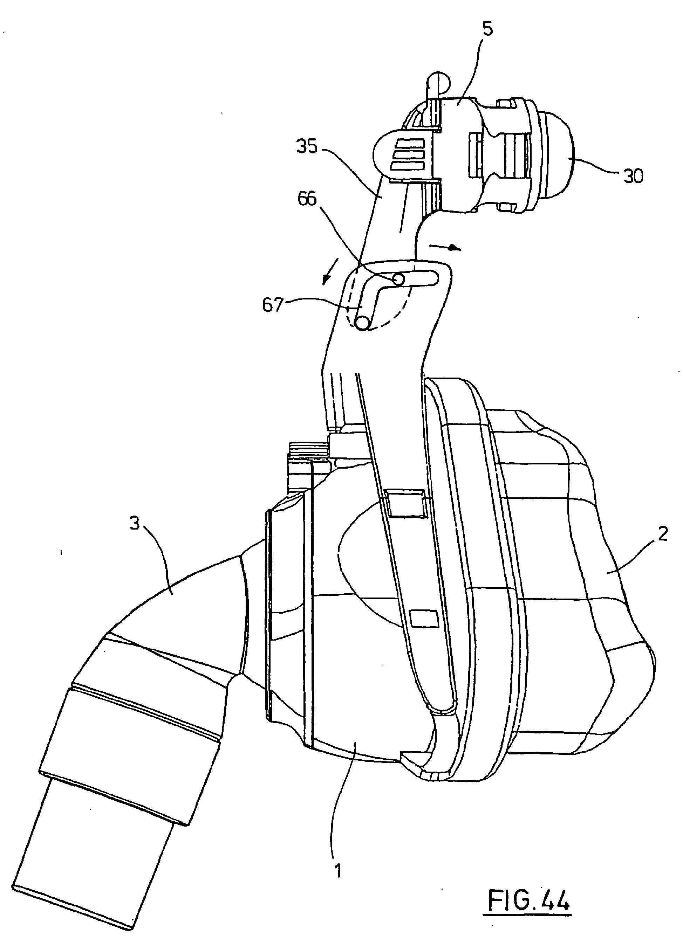

Gemäß dem Beispiel in Fig. 44 weist der Schaft (35) mindestens ein Führungselement (66) auf, das entlang einer gekrümmt verlaufenden Führungsbahn (67) verschieblich ist. Das Führungselement (66) kann beispielsweise als Zapfen und die Führungsbahn (67) als Nut ausgebildet sein. Es wird hierdurch eine reproduzierbare Zwangsführung für die Stirnstütze (5) bereitgestellt.

## Patentansprüche

1. Vorrichtung zur Beatmung, die als mindestens ein Teil eines Benutzerinterfaces ausgebildet ist, und bei der mindestens zwei Komponenten des Benutzerinterfaces durch eine manuell lösbare Arretierung miteinander verbunden sind, wobei das Benutzerinterface als Atemmaske ausgebildet ist, die eine Stirnstütze (5) aufweist, die mit einer Verstellung zur Veränderung der Stirnstütze (5) relativ zu einem Maskengrundkörper (1) versehen ist und bei der die lösbare Arretierung eine lineare gerastete Feinverstellung (17) mit einem Tragsteg (28) umfaßt, der eine Sockelplatte (29) der Stirnseite (5) haltert, wobei an der Sockelplatte (29) ein Stirnpolster (30) befestigt ist, wobei sich der Tragsteg (28) ausgehend von der Sockelplatte (29) in eine dem Stirnpolster (30) abgewandte Richtung erstreckt, wobei zur Führung des Tragsteges (28) ein Schaft (35) mit einem Tragprofil (53) versehen ist, wobei die Arretierung mindestens eine Rastung umfasst und wobei die Rastung durch einen manuellen Druck von einem Gegenprofil trennbar angeordnet ist, **dadurch gekennzeichnet, dass** ein Anschlag (55) im Schaft (35) der Begrenzung des Verstellweges des Tragsteges (28) innerhalb der Feinverstellung (17) dient und dass das Tragprofil (53) in eine Führungsausnehmung (54) des Tragsteges (28) eingreift und dass die Rastung mit einer Einführanschrägung versehen ist und die Rastung mindestens eine Rastnase aufweist.

2. Vorrichtung nach Anspruch 1, **dadurch gekennzeichnet, dass** sowohl eine Feinverstellung (17) mit einer feinen Rastung als auch eine Grobverstellung (16) in Form einer Umsteckbarkeit der Stirnstütze (5) im Maskenkörper (1) realisiert ist.

3. Vorrichtung nach Anspruch 2, **dadurch gekennzeichnet, dass** die Stirnstütze (5) wahlweise in jeweils mindestens einer von mehreren Steckpositionen im Maskenkörper (1) als eine Grobverstellung (16) positionierbar ist.

4. Vorrichtung nach einem der Ansprüche 1 bis 3, **dadurch gekennzeichnet, dass** der Maskengrundkörper (1) einen Adapter zur Verbindung mit der Stirnstütze (5) aufweist und dass der Adapter zur Aufnahme mindestens eines unterschiedlich zur Stirnstütze (5) gestalteten Bauteiles ausgebildet ist.

5. Vorrichtung nach einem der Ansprüche 1 bis 4, **dadurch gekennzeichnet, dass** die Sockelplatte (29) der Stirnstütze (5) eine Zentrierung zur Ausrichtung des Stirnpolsters (30) aufweist.

6. Vorrichtung nach einem der Ansprüche 2 bis 3, **dadurch gekennzeichnet, dass** die Grobverstellung (16) durch einen manuellen Druck entriegelbar ausgebildet ist.

7. Vorrichtung nach einem der Ansprüche 1 bis 6, **dadurch gekennzeichnet, dass** die Vorrichtung gewölbt konturierte Bedienflächen an der Feineinstellung (17) zur Aufbringung von Bedienkräften aufweist.

8. Vorrichtung nach einem der Ansprüche 1 bis 7, **dadurch gekennzeichnet, dass** mindestens eines der Bauteile aus einem mit Versteifungsrippen versehenen dünnen Material ausgebildet ist.

9. Vorrichtung nach Anspruch 7, **dadurch gekennzeichnet, dass** die gewölbten Bedienflächen Griffmulden eine Leitfunktion bereitstellen.

10. Vorrichtung nach einem der Ansprüche 1 bis 9, **dadurch gekennzeichnet, dass** der Maskengrundkörper (1) mindestens eine durch einen dünnwandigen dreidimensionalen Materialverlauf ausgebildete Halterungsstruktur aufweist.

11. Vorrichtung nach einem der Ansprüche 2 oder 3, **dadurch gekennzeichnet, dass** die Grobverstellung (16) im Bereich eines Überganges des Schaftes (35) der Stirnstütze (5) zum Maskengrundkörper (1) angeordnet ist.

12. Vorrichtung nach einem der Ansprüche 1 bis 11, **dadurch gekennzeichnet, dass** die Feinverstellung (17) im Bereich eines Überganges des Schaftes (35) zur Sockelplatte (29) angeordnet ist.

13. Vorrichtung nach einem der Ansprüche 2 bis 12, **dadurch gekennzeichnet, dass** die Grobverstellung (16) den im Bereich von Halterungsausnehmungen (36) des Maskenkörpers (1) umsteckbaren Schaft (35) der Stirnstütze (5) sowie eine durch federnde Befestigungslaschen (25) bereitgestellte Arretierung umfasst.

14. Vorrichtung nach einem der Ansprüche 1 bis 13, **dadurch gekennzeichnet, dass** der ist Tragsteg (28) der Sockelplatte (29) aus der Führungsausnehmung (54) des Schaftes (35) herausziehbar und die Arretierung der Feinverstellung (17) Bedienlaschen (33) umfaßt.

15. Vorrichtung nach einem der Ansprüche 13 oder 14, **dadurch gekennzeichnet, dass** die Befestigungslaschen (25) und die Bedienlaschen (33) in einer Längsrichtung des Schaftes (35) hintereinander angeordnet sind.

## Claims

1. A respiratory device, which is constructed as at least part of a user interface and in which at least two components of the user interface are connected to one another by a manually releasable locking mechanism, wherein the user interface is constructed as a respiratory mask, which has a forehead connection piece (5) which is provided with an adjustment means for altering the forehead connection piece (5) relative to a mask base body (1) and in which the releasable locking mechanism comprises a linear detented fine adjustment means (17) with a supporting web (28) which holds a base plate (29) of the forehead side (5), wherein a forehead pad (30) is fastened to the base plate (29), wherein the supporting web (28), starting from the base plate (29), extends in a direction away from the forehead pad (30), wherein a shaft (35) with a supporting profile (53) is provided to guide the supporting web (28), wherein the locking mechanism comprises at least one detent and wherein the detent is arranged to be separable from a counter profile by manual pressure, **characterised in that** a stop (55) in the shaft (35) serves to delimit the adjustment path of the supporting web (28) within the fine adjustment means (17), and **in that** the supporting profile (53) engages in a guide recess (54) in the supporting web (28) and **in that** the detent is provided with a chamfered lead-in portion and the detent has at least one detent projection.

2. A device according to Claim 1, **characterised in that** both a fine adjustment means (17) with a fine detent as well as a coarse adjustment means (16) in the form of a repluggability of the forehead connection-piece (5) in the mask body (1) is realised.

3. A device according to Claim 2, **characterised in that** the forehead connection-piece (5) can be optionally positioned in each case in at least one of a plurality of plug-in positions in the mask body (1) as a coarse adjustment means (16).

4. A device according to one of Claims 1 to 3, **characterised in that** the mask base body (1) has an adapter for connection to the forehead connection-piece (5) and **in that** the adapter is constructed to receive at least one component which is of a different design from the forehead connection-piece (5).

5. A device according to one of Claims 1 to 4, **characterised in that** the base plate (29) of the forehead connection-piece (5) has a centring means for aligning the forehead pad (30).

6. A device according to one of Claims 2 to 3, **characterised in that** the coarse adjustment means (16) is designed to be unlockable by manual pressure.

7. A device according to one of Claims 1 to 6, **characterised in that** the device has curvedly contoured operating surfaces on the fine adjustment means (17) for the application of operating forces.

8. A device according to one of Claims 1 to 7, **characterised in that** at least one of the components is constructed from a thin material provided with reinforcing ribs.

9. A device according to Claim 7, **characterised in that** the curved operating surfaces provide grip recesses with a control function.

10. A device according to one of Claims 1 to 9, **characterised in that** the mask base body (1) has at least one holding structure formed by a thin-walled three-dimensional material progression.

11. A device according to one of Claims 2 or 3, **characterised in that** the coarse adjustment means (16) is arranged in the region of a transition of the shaft (35) of the forehead connection-piece (5) into the mask base body (1).

12. A device according to one of Claims 1 to 11, **characterised in that** the fine adjustment means (17) is arranged in the region of a transition of the shaft (35) into the base plate (29).

13. A device according to one of Claims 2 to 12, **characterised in that** the coarse adjustment means (16) comprises the shaft (35) of the forehead connection-piece (5), which shaft is repluggable in the region of the holding recesses (36) in the mask body (1), and a locking mechanism provided by resilient fastening lugs (25).

14. A device according to one of Claims 1 to 13, **characterised in that** the supporting web (28) of the base plate (29) can be pulled out of the guide recess (54) of the shaft (35) and the locking mechanism of the fine adjustment means (17) comprises operating lugs (33).

15. A device according to one of Claims 13 or 14, **characterised in that** the fastening lugs (25) and the operating lugs (33) are arranged in succession in a longitudinal direction of the shaft (35).

## Revendications

1. Dispositif respiratoire, qui est conçu en tant que partie d'une interface utilisateur et dans lequel au moins deux composants de l'interface utilisateur sont reliés ensemble par un système de blocage amovible à la main, sachant que l'interface utilisateur est réalisée sous la forme d'un masque respiratoire, qui est doté d'un support frontal (5) pourvu d'un réglage dudit support frontal (5) par rapport à un corps de base du masque (1), et dans lequel le système de blocage amovible comprend un réglage fin (17) linéaire avec une patte de support (28) qui maintient une plaque de socle (29) du support frontal (5), sachant qu'un rembourrage frontal (30) est fixé sur la plaque de socle (29), la patte de support (28) s'étendant dans une direction opposée au rembourrage frontal (30), sachant que, pour le guidage de la patte de support (28), une tige (35) est pourvue d'un profil (53), le système de blocage comprenant au moins un crantage, et le crantage étant agencé de sorte à pouvoir être séparé d'un contreprofilé pat une pression manuelle, **caractérisé en ce qu'une** butée (55), dans la tige (35), sert à limiter la course de la patte de support (28) du réglage fin et que le profilé de support (53) s'engage dans un évidement de guidage (54) de la patte de support (28) et que le crantage est pourvu d'un chanfrein d'insertion et qu'il présente au moins un mentonnet d'arrêt.

2. Dispositif selon la revendication 1, **caractérisé en ce que** aussi bien un réglage fin (17) avec un fin crantage qu'un réglage grossier (16) sont réalisés sous la forme d'un système permettant le repositionnement du support frontal (5) dans le corps de base du masque (1).

3. Dispositif selon la revendication 2,
**caractérisé en ce que** le support frontal (5) peut, au choix, être positionné, dans le corps de base du masque (1), dans au moins l'une de plusieurs positions enfichées, en tant que réglage grossier (16).

4. Dispositif selon l'une des revendications 1 à 3,
**caractérisé en ce que** le corps de base du masque (1) est doté d'un adaptateur pour le raccordement au support frontal (5) et q u e ledit adaptateur est configuré pour recevoir au moins un composant dont la conception diffère de celle du support frontal (5).

5. Dispositif selon l'une des revendications 1 à 4,
**caractérisé en ce que** la plaque de socle (29) du support frontal (5) est dotée d'un centrage pour le placement du rembourrage frontal (30).

6. Dispositif selon l'une des revendications 2 à 3,
**caractérisé en ce que** le réglage grossier (16) peut être déverrouillé par une pression manuelle.

7. Dispositif selon l'une des revendications 1 à 6,
**caractérisé en ce que,** pour l'application des force de commande, le dispositif est doté, sur le réglage fin, de surface de commande aux contour courbes.

8. Dispositif selon l'une des revendications 1 à 7,
**caractérisé en ce que** l'un des composants, au moins, consiste en matériau de faible épaisseur, pourvu de nervures de raidissage.

9. Dispositif selon la revendication 7,
**caractérisé en ce que** les surfaces de commande courbes, poignées noyées, assument une fonction directrice.

10. Dispositif selon l'une des revendications 1 à 9,
**caractérisé en ce que** le corps de base du masque (1) présente au moins une structure de support qui est formée par la contexture tridimensionnelle du matériau de faible épaisseur.

11. Dispositif selon l'une des revendications 2 ou 3,
**caractérisé en ce que** le réglage grossier (16) est aménagé dans la zone de transition entre la tige (35) du support frontal (5) au corps de base du masque (1).

12. Dispositif selon l'une des revendications 1 à 11,
**caractérisé en ce que** le réglage fin (17) est aménagé dans une zone de transition entre la tige (35) et la plaque de socle (29).

13. Dispositif selon l'une des revendications 2 à 12,
**caractérisé en ce que** le réglage grossier (16) comprend la tige (35) du support frontal (5) pouvant être repositionnée dans des évidements (36) du corps de base du masque (1), ainsi qu'un système de blocage qui est formé par des pattes de fixations (25) élastiques.

14. Dispositif selon l'une des revendications 1 à 13,
**caractérisé en ce que** la patte de support (28) de la plaque de socle (29) peut être extraite de l'évidement de guidage (54) et que le blocage du réglage fin (17) comprend des pattes de commande (33).

15. Dispositif selon l'une des revendications 13 ou 14,
**caractérisé en ce que** les pattes de fixation (25) et les pattes de commande (33) sont disposées les unes derrière les autres dans la direction longitudinale de la tige (35).
